(19) Europäisches Patentamt
European Patent Office
Office européen des brevets

(11) EP 3 473 272 A1

(12) EUROPEAN PATENT APPLICATION

(43) Date of publication:
**24.04.2019 Bulletin 2019/17**

(21) Application number: **18211716.8**

(22) Date of filing: **28.03.2014**

(51) Int Cl.:
***A61K 39/395*** *(2006.01)* ***A61K 48/00*** *(2006.01)*
***C07K 16/18*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.03.2013 US 201361806687 P**

(62) Document number(s) of the earlier application(s) in accordance with Art. 76 EPC:
**14722916.5 / 2 978 451**

(71) Applicant: **Alexion Pharmaceuticals, Inc.**
**Boston, MA 02210 (US)**

(72) Inventors:
- **HUNTER, Jeffrey, W.**
**New Britain, CT 06053 (US)**
- **TAMBURINI, Paul, P.**
**Kensington, CT 06037 (US)**

(74) Representative: **J A Kemp**
**14 South Square**
**Gray's Inn**
**London WC1R 5JJ (GB)**

Remarks:
This application was filed on 11-12-2018 as a divisional application to the application mentioned under INID code 62.

# (54) COMPOSITIONS AND METHODS FOR INCREASING THE SERUM HALF-LIFE OF A THERAPEUTIC AGENT TARGETING COMPLEMENT C5

(57) The disclosure features compositions and methods for increasing the half-life of a therapeutic agent (e.g., a C5 antagonist) in the serum of a subject (e.g., a human). Also featured are compositions and methods for: (i) decreasing the frequency by which a therapeutically effective amount of a therapeutic agent must be administered to a human having, suspected of having, or at risk for developing, a medical condition for which the therapeutic agent is effective and (ii) decreasing the dosage of the therapeutic agent required for therapeutic efficacy in a human having, suspected of having, or at risk for developing, a medical condition for which the therapeutic agent is effective. The methods include reducing the serum concentration of the antigen to which the therapeutic agent binds.

EP 3 473 272 A1

SEQUENCE LISTING

<110> ALEXION PHARMACEUTICALS, INC.

<120> COMPOSITIONS AND METHODS FOR INCREASING THE SERUM HALF-LIFE OF A THERAPEUTIC AGENT TARGETING COMPLEMENT C5

<130> AXJ-171EP

<140>
<141>

<150> PCT/US2014/032209
<151> 2014-03-28

<150> 61/806,687
<151> 2013-03-29

<160> 2

<170> PatentIn version 3.5

<210> 1
<211> 5444
<212> DNA
<213> Homo sapiens

<400> 1
ctacctccaa ccatgggcct tttgggaata ctttgttttt taatcttcct ggggaaaacc 60

tggggacagg agcaaacata tgtcatttca gcaccaaaaa tattccgtgt tggagcatct 120

gaaaatattg tgattcaagt ttatggatac actgaagcat ttgatgcaac aatctctatt 180

aaaagttatc ctgataaaaa atttagttac tcctcaggcc atgttcattt atcctcagag 240

aataaattcc aaaactctgc aatcttaaca atacaaccaa aacaattgcc tggaggacaa 300

aacccagttt cttatgtgta tttggaagtt gtatcaaagc atttttcaaa atcaaaaaga 360

atgccaataa cctatgacaa tggatttctc ttcattcata cagacaaacc tgtttatact 420

ccagaccagt cagtaaaagt tagagtttat tcgttgaatg acgacttgaa gccagccaaa 480

agagaaactg tcttaacctt catagatcct gaaggatcag aagttgacat ggtagaagaa 540

attgatcata ttggaattat ctcttttcct gacttcaaga ttccgtctaa tcctagatat 600

ggtatgtgga cgatcaaggc taaatataaa gaggactttt caacaactgg aaccgcatat 660

tttgaagtta aagaatatgt cttgccacat ttttctgtct caatcgagcc agaatataat 720

ttcattggtt acaagaactt taagaatttt gaaattacta taaaagcaag atatttttat 780

aataaagtag tcactgaggc tgacgtttat atcacatttg gaataagaga agacttaaaa 840

gatgatcaaa aagaaatgat gcaaacagca atgcaaaaca caatgttgat aaatggaatt 900

gctcaagtca catttgattc tgaaacagca gtcaaagaac tgtcatacta cagtttagaa 960

gatttaaaca acaagtacct ttatattgct gtaacagtca tagagtctac aggtggattt 1020

tctgaagagg cagaaatacc tggcatcaaa tatgtcctct ctccctacaa actgaatttg 1080

gttgctactc ctcttttcct gaagcctggg attccatatc ccatcaaggt gcaggttaaa 1140

gattcgcttg accagttggt aggaggagtc ccagtaatac tgaatgcaca aacaattgat 1200

gtaaaccaag agacatctga cttggatcca agcaaaagtg taacacgtgt tgatgatgga 1260

gtagcttcct ttgtgcttaa tctcccatct ggagtgacgg tgctggagtt taatgtcaaa 1320

actgatgctc cagatcttcc agaagaaaat caggccaggg aaggttaccg agcaatagca 1380

tactcatctc tcagccaaag ttacctttat attgattgga ctgataacca taaggctttg 1440

ctagtgggag aacatctgaa tattattgtt acccccaaaa gcccatatat tgacaaaata 1500

actcactata attacttgat tttatccaag ggcaaaatta tccattttgg cacgagggag 1560

aaattttcag atgcatctta tcaaagtata aacattccag taacacagaa catggttcct 1620

tcatcccgac ttctggtcta ttatatcgtc acaggagaac agacagcaga attagtgtct 1680

gattcagtct ggttaaatat tgaagaaaaa tgtggcaacc agctccaggt tcatctgtct 1740

cctgatgcag atgcatattc tccaggccaa actgtgtctc ttaatatggc aactggaatg 1800

gattcctggg tggcattagc agcagtggac agtgctgtgt atggagtcca aagaggagcc 1860

aaaaagccct tggaaagagt atttcaattc ttagagaaga gtgatctggg ctgtggggca 1920

ggtggtggcc tcaacaatgc caatgtgttc cacctagctg gacttacctt cctcactaat 1980

gcaaatgcag atgactccca agaaaatgat gaaccttgta aagaaattct caggccaaga 2040

agaacgctgc aaaagaagat agaagaaata gctgctaaat ataaacattc agtagtgaag 2100

aaatgttgtt acgatggagc ctgcgttaat aatgatgaaa cctgtgagca gcgagctgca 2160

cggattagtt tagggccaag atgcatcaaa gctttcactg aatgttgtgt cgtcgcaagc 2220

cagctccgtg ctaatatctc tcataaagac atgcaattgg gaaggctaca catgaagacc 2280

ctgttaccag taagcaagcc agaaattcgg agttattttc cagaaagctg gttgtgggaa 2340

gttcatcttg ttcccagaag aaaacagttg cagtttgccc tacctgattc tctaaccacc 2400

tgggaaattc aaggcattgg catttcaaac actggtatat gtgttgctga tactgtcaag 2460

gcaaaggtgt tcaaagatgt cttcctggaa atgaatatac catattctgt tgtacgagga 2520

gaacagatcc aattgaaagg aactgtttac aactatagga cttctgggat gcagttctgt 2580

gttaaaatgt ctgctgtgga gggaatctgc acttcggaaa gcccagtcat tgatcatcag 2640

ggcacaaagt cctccaaatg tgtgcgccag aaagtagagg gctcctccag tcacttggtg 2700

acattcactg tgcttcctct ggaaattggc cttcacaaca tcaatttttc actggagact 2760

tggtttggaa aagaaatctt agtaaaaaca ttacgagtgg tgccagaagg tgtcaaaagg 2820

gaaagctatt ctggtgttac tttggatcct aggggtattt atggtaccat tagcagacga 2880

aaggagttcc catacaggat acccttagat ttggtcccca aaacagaaat caaaaggatt 2940

ttgagtgtaa aaggactgct tgtaggtgag atcttgtctg cagttctaag tcaggaaggc 3000
atcaatatcc taacccacct ccccaaaggg agtgcagagg cggagctgat gagcgttgtc 3060
ccagtattct atgtttttca ctacctggaa acaggaaatc attggaacat ttttcattct 3120
gacccattaa ttgaaaagca gaaactgaag aaaaaattaa aagaagggat gttgagcatt 3180
atgtcctaca gaaatgctga ctactcttac agtgtgtgga agggtggaag tgctagcact 3240
tggttaacag cttttgcttt aagagtactt ggacaagtaa ataaatacgt agagcagaac 3300
caaaattcaa tttgtaattc tttattgtgg ctagttgaga attatcaatt agataatgga 3360
tctttcaagg aaaattcaca gtatcaacca ataaaattac agggtacctt gcctgttgaa 3420
gcccgagaga acagcttata tcttacagcc tttactgtga ttggaattag aaaggctttc 3480
gatatatgcc ccctggtgaa aatcgacaca gctctaatta aagctgacaa ctttctgctt 3540
gaaaatacac tgccagccca gagcaccttt acattggcca tttctgcgta tgctctttcc 3600
ctgggagata aaactcaccc acagtttcgt tcaattgttt cagctttgaa gagagaagct 3660
ttggttaaag gtaatccacc catttatcgt tttggaaag acaatcttca gcataaagac 3720
agctctgtac ctaacactgg tacggcacgt atggtagaaa caactgccta tgctttactc 3780
accagtctga acttgaaaga tataaattat gttaacccag tcatcaaatg gctatcagaa 3840
gagcagaggt atggaggtgg cttttattca acccaggaca ccatcaatgc cattgagggc 3900
ctgacggaat attcactcct ggttaaacaa ctccgcttga gtatggacat cgatgtttct 3960
tacaagcata aaggtgcctt acataattat aaaatgacag acaagaattt ccttgggagg 4020
ccagtagagg tgcttctcaa tgatgacctc attgtcagta caggatttgg cagtggcttg 4080
gctacagtac atgtaacaac tgtagttcac aaaaccagta cctctgagga agtttgcagc 4140
ttttatttga aaatcgatac tcaggatatt gaagcatccc actacagagg ctacggaaac 4200
tctgattaca aacgcatagt agcatgtgcc agctacaagc ccagcaggga agaatcatca 4260
tctggatcct ctcatgcggt gatggacatc tccttgccta ctggaatcag tgcaaatgaa 4320
gaagacttaa aagcccttgt ggaaggggtg gatcaactat tcactgatta ccaaatcaaa 4380
gatggacatg ttattctgca actgaattcg attccctcca gtgatttcct ttgtgtacga 4440
ttccggatat ttgaactctt tgaagttggg tttctcagtc ctgccacttt cacagtttac 4500
gaataccaca gaccagataa acagtgtacc atgttttata gcacttccaa tatcaaaatt 4560
cagaaagtct gtgaaggagc cgcgtgcaag tgtgtagaag ctgattgtgg gcaaatgcag 4620
gaagaattgg atctgacaat ctctgcagag acaagaaaac aaacagcatg taaaccagag 4680
attgcatatg cttataaagt tagcatcaca tccatcactg tagaaaatgt tttgtcaag 4740
tacaaggcaa cccttctgga tatctacaaa actggggaag ctgttgctga gaaagactct 4800
gagattacct tcattaaaaa ggtaacctgt actaacgctg agctggtaaa aggaagacag 4860

```
tacttaatta tgggtaaaga agccctccag ataaaataca atttcagttt caggtacatc      4920

taccctttag attccttgac ctggattgaa tactggccta gagacacaac atgttcatcg      4980

tgtcaagcat ttttagctaa tttagatgaa tttgccgaag atatcttttt aaatggatgc      5040

taaaattcct gaagttcagc tgcatacagt ttgcacttat ggactcctgt tgttgaagtt      5100

cgtttttttg ttttcttctt tttttaaaca ttcatagctg gtcttatttg taaagctcac      5160

tttacttaga attagtggca cttgctttta ttagagaatg atttcaaatg ctgtaacttt      5220

ctgaaataac atggccttgg agggcatgaa gacagatact cctccaaggt tattggacac      5280

cggaaacaat aaattggaac acctcctcaa acctaccact caggaatgtt tgctggggcc      5340

gaaagaacag tccattgaaa gggagtatta caaaaacatg gcctttgctt gaaagaaaat      5400

accaaggaac aggaaactga tcattaaagc ctgagtttgc tttc                      5444


<210> 2
<211> 1676
<212> PRT
<213> Homo sapiens

<400> 2
Met Gly Leu Leu Gly Ile Leu Cys Phe Leu Ile Phe Leu Gly Lys Thr
1               5                   10                  15


Trp Gly Gln Glu Gln Thr Tyr Val Ile Ser Ala Pro Lys Ile Phe Arg
            20                  25                  30


Val Gly Ala Ser Glu Asn Ile Val Ile Gln Val Tyr Gly Tyr Thr Glu
        35                  40                  45


Ala Phe Asp Ala Thr Ile Ser Ile Lys Ser Tyr Pro Asp Lys Lys Phe
    50                  55                  60


Ser Tyr Ser Ser Gly His Val His Leu Ser Ser Glu Asn Lys Phe Gln
65                  70                  75                  80


Asn Ser Ala Ile Leu Thr Ile Gln Pro Lys Gln Leu Pro Gly Gly Gln
                85                  90                  95


Asn Pro Val Ser Tyr Val Tyr Leu Glu Val Val Ser Lys His Phe Ser
            100                 105                 110


Lys Ser Lys Arg Met Pro Ile Thr Tyr Asp Asn Gly Phe Leu Phe Ile
        115                 120                 125


His Thr Asp Lys Pro Val Tyr Thr Pro Asp Gln Ser Val Lys Val Arg
    130                 135                 140
```

Val Tyr Ser Leu Asn Asp Asp Leu Lys Pro Ala Lys Arg Glu Thr Val
145                 150                 155                 160

Leu Thr Phe Ile Asp Pro Glu Gly Ser Glu Val Asp Met Val Glu Glu
                165                 170                 175

Ile Asp His Ile Gly Ile Ile Ser Phe Pro Asp Phe Lys Ile Pro Ser
            180                 185                 190

Asn Pro Arg Tyr Gly Met Trp Thr Ile Lys Ala Lys Tyr Lys Glu Asp
        195                 200                 205

Phe Ser Thr Thr Gly Thr Ala Tyr Phe Glu Val Lys Glu Tyr Val Leu
    210                 215                 220

Pro His Phe Ser Val Ser Ile Glu Pro Glu Tyr Asn Phe Ile Gly Tyr
225                 230                 235                 240

Lys Asn Phe Lys Asn Phe Glu Ile Thr Ile Lys Ala Arg Tyr Phe Tyr
                245                 250                 255

Asn Lys Val Val Thr Glu Ala Asp Val Tyr Ile Thr Phe Gly Ile Arg
            260                 265                 270

Glu Asp Leu Lys Asp Asp Gln Lys Glu Met Met Gln Thr Ala Met Gln
        275                 280                 285

Asn Thr Met Leu Ile Asn Gly Ile Ala Gln Val Thr Phe Asp Ser Glu
    290                 295                 300

Thr Ala Val Lys Glu Leu Ser Tyr Tyr Ser Leu Glu Asp Leu Asn Asn
305                 310                 315                 320

Lys Tyr Leu Tyr Ile Ala Val Thr Val Ile Glu Ser Thr Gly Gly Phe
                325                 330                 335

Ser Glu Glu Ala Glu Ile Pro Gly Ile Lys Tyr Val Leu Ser Pro Tyr
            340                 345                 350

Lys Leu Asn Leu Val Ala Thr Pro Leu Phe Leu Lys Pro Gly Ile Pro
        355                 360                 365

Tyr Pro Ile Lys Val Gln Val Lys Asp Ser Leu Asp Gln Leu Val Gly
    370                 375                 380

Gly Val Pro Val Thr Leu Asn Ala Gln Thr Ile Asp Val Asn Gln Glu
385                 390                 395                 400

Thr Ser Asp Leu Asp Pro Ser Lys Ser Val Thr Arg Val Asp Asp Gly
405 410 415

Val Ala Ser Phe Val Leu Asn Leu Pro Ser Gly Val Thr Val Leu Glu
420 425 430

Phe Asn Val Lys Thr Asp Ala Pro Asp Leu Pro Glu Glu Asn Gln Ala
435 440 445

Arg Glu Gly Tyr Arg Ala Ile Ala Tyr Ser Ser Leu Ser Gln Ser Tyr
450 455 460

Leu Tyr Ile Asp Trp Thr Asp Asn His Lys Ala Leu Leu Val Gly Glu
465 470 475 480

His Leu Asn Ile Ile Val Thr Pro Lys Ser Pro Tyr Ile Asp Lys Ile
485 490 495

Thr His Tyr Asn Tyr Leu Ile Leu Ser Lys Gly Lys Ile Ile His Phe
500 505 510

Gly Thr Arg Glu Lys Phe Ser Asp Ala Ser Tyr Gln Ser Ile Asn Ile
515 520 525

Pro Val Thr Gln Asn Met Val Pro Ser Ser Arg Leu Leu Val Tyr Tyr
530 535 540

Ile Val Thr Gly Glu Gln Thr Ala Glu Leu Val Ser Asp Ser Val Trp
545 550 555 560

Leu Asn Ile Glu Glu Lys Cys Gly Asn Gln Leu Gln Val His Leu Ser
565 570 575

Pro Asp Ala Asp Ala Tyr Ser Pro Gly Gln Thr Val Ser Leu Asn Met
580 585 590

Ala Thr Gly Met Asp Ser Trp Val Ala Leu Ala Ala Val Asp Ser Ala
595 600 605

Val Tyr Gly Val Gln Arg Gly Ala Lys Lys Pro Leu Glu Arg Val Phe
610 615 620

Gln Phe Leu Glu Lys Ser Asp Leu Gly Cys Gly Ala Gly Gly Gly Leu
625 630 635 640

Asn Asn Ala Asn Val Phe His Leu Ala Gly Leu Thr Phe Leu Thr Asn

645 650 655

Ala Asn Ala Asp Asp Ser Gln Glu Asn Asp Glu Pro Cys Lys Glu Ile
660 665 670

Leu Arg Pro Arg Arg Thr Leu Gln Lys Lys Ile Glu Glu Ile Ala Ala
675 680 685

Lys Tyr Lys His Ser Val Val Lys Lys Cys Cys Tyr Asp Gly Ala Cys
690 695 700

Val Asn Asn Asp Glu Thr Cys Glu Gln Arg Ala Ala Arg Ile Ser Leu
705 710 715 720

Gly Pro Arg Cys Ile Lys Ala Phe Thr Glu Cys Cys Val Val Ala Ser
725 730 735

Gln Leu Arg Ala Asn Ile Ser His Lys Asp Met Gln Leu Gly Arg Leu
740 745 750

His Met Lys Thr Leu Leu Pro Val Ser Lys Pro Glu Ile Arg Ser Tyr
755 760 765

Phe Pro Glu Ser Trp Leu Trp Glu Val His Leu Val Pro Arg Arg Lys
770 775 780

Gln Leu Gln Phe Ala Leu Pro Asp Ser Leu Thr Thr Trp Glu Ile Gln
785 790 795 800

Gly Val Gly Ile Ser Asn Thr Gly Ile Cys Val Ala Asp Thr Val Lys
805 810 815

Ala Lys Val Phe Lys Asp Val Phe Leu Glu Met Asn Ile Pro Tyr Ser
820 825 830

Val Val Arg Gly Glu Gln Ile Gln Leu Lys Gly Thr Val Tyr Asn Tyr
835 840 845

Arg Thr Ser Gly Met Gln Phe Cys Val Lys Met Ser Ala Val Glu Gly
850 855 860

Ile Cys Thr Ser Glu Ser Pro Val Ile Asp His Gln Gly Thr Lys Ser
865 870 875 880

Ser Lys Cys Val Arg Gln Lys Val Glu Gly Ser Ser Ser His Leu Val
885 890 895

```
Thr Phe Thr Val Leu Pro Leu Glu Ile Gly Leu His Asn Ile Asn Phe
            900                 905                 910

Ser Leu Glu Thr Trp Phe Gly Lys Glu Ile Leu Val Lys Thr Leu Arg
        915                 920                 925

Val Val Pro Glu Gly Val Lys Arg Glu Ser Tyr Ser Gly Val Thr Leu
    930                 935                 940

Asp Pro Arg Gly Ile Tyr Gly Thr Ile Ser Arg Arg Lys Glu Phe Pro
945                 950                 955                 960

Tyr Arg Ile Pro Leu Asp Leu Val Pro Lys Thr Glu Ile Lys Arg Ile
                965                 970                 975

Leu Ser Val Lys Gly Leu Leu Val Gly Glu Ile Leu Ser Ala Val Leu
            980                 985                 990

Ser Gln Glu Gly Ile Asn Ile Leu  Thr His Leu Pro Lys  Gly Ser Ala
        995                 1000                1005

Glu Ala  Glu Leu Met Ser Val  Val Pro Val Phe Tyr  Val Phe His
    1010                 1015                 1020

Tyr Leu  Glu Thr Gly Asn His  Trp Asn Ile Phe His  Ser Asp Pro
    1025                 1030                 1035

Leu Ile  Glu Lys Gln Lys Leu  Lys Lys Lys Leu Lys  Glu Gly Met
    1040                 1045                 1050

Leu Ser  Ile Met Ser Tyr Arg  Asn Ala Asp Tyr Ser  Tyr Ser Val
    1055                 1060                 1065

Trp Lys  Gly Gly Ser Ala Ser  Thr Trp Leu Thr Ala  Phe Ala Leu
    1070                 1075                 1080

Arg Val  Leu Gly Gln Val Asn  Lys Tyr Val Glu Gln  Asn Gln Asn
    1085                 1090                 1095

Ser Ile  Cys Asn Ser Leu Leu  Trp Leu Val Glu Asn  Tyr Gln Leu
    1100                 1105                 1110

Asp Asn  Gly Ser Phe Lys Glu  Asn Ser Gln Tyr Gln  Pro Ile Lys
    1115                 1120                 1125

Leu Gln  Gly Thr Leu Pro Val  Glu Ala Arg Glu Asn  Ser Leu Tyr
    1130                 1135                 1140
```

```
Leu Thr  Ala Phe Thr Val Ile  Gly Ile Arg Lys Ala  Phe Asp Ile
    1145                 1150                 1155

Cys Pro  Leu Val Lys Ile Asp  Thr Ala Leu Ile Lys  Ala Asp Asn
    1160                 1165                 1170

Phe Leu  Leu Glu Asn Thr Leu  Pro Ala Gln Ser Thr  Phe Thr Leu
    1175                 1180                 1185

Ala Ile  Ser Ala Tyr Ala Leu  Ser Leu Gly Asp Lys  Thr His Pro
    1190                 1195                 1200

Gln Phe  Arg Ser Ile Val Ser  Ala Leu Lys Arg Glu  Ala Leu Val
    1205                 1210                 1215

Lys Gly  Asn Pro Pro Ile Tyr  Arg Phe Trp Lys Asp  Asn Leu Gln
    1220                 1225                 1230

His Lys  Asp Ser Ser Val Pro  Asn Thr Gly Thr Ala  Arg Met Val
    1235                 1240                 1245

Glu Thr  Thr Ala Tyr Ala Leu  Leu Thr Ser Leu Asn  Leu Lys Asp
    1250                 1255                 1260

Ile Asn  Tyr Val Asn Pro Val  Ile Lys Trp Leu Ser  Glu Glu Gln
    1265                 1270                 1275

Arg Tyr  Gly Gly Gly Phe Tyr  Ser Thr Gln Asp Thr  Ile Asn Ala
    1280                 1285                 1290

Ile Glu  Gly Leu Thr Glu Tyr  Ser Leu Leu Val Lys  Gln Leu Arg
    1295                 1300                 1305

Leu Ser  Met Asp Ile Asp Val  Ser Tyr Lys His Lys  Gly Ala Leu
    1310                 1315                 1320

His Asn  Tyr Lys Met Thr Asp  Lys Asn Phe Leu Gly  Arg Pro Val
    1325                 1330                 1335

Glu Val  Leu Leu Asn Asp Asp  Leu Ile Val Ser Thr  Gly Phe Gly
    1340                 1345                 1350

Ser Gly  Leu Ala Thr Val His  Val Thr Thr Val Val  His Lys Thr
    1355                 1360                 1365

Ser Thr  Ser Glu Glu Val Cys  Ser Phe Tyr Leu Lys  Ile Asp Thr
    1370                 1375                 1380
```

Gln Asp Ile Glu Ala Ser His Tyr Arg Gly Tyr Gly Asn Ser Asp
1385 1390 1395

Tyr Lys Arg Ile Val Ala Cys Ala Ser Tyr Lys Pro Ser Arg Glu
1400 1405 1410

Glu Ser Ser Ser Gly Ser Ser His Ala Val Met Asp Ile Ser Leu
1415 1420 1425

Pro Thr Gly Ile Ser Ala Asn Glu Glu Asp Leu Lys Ala Leu Val
1430 1435 1440

Glu Gly Val Asp Gln Leu Phe Thr Asp Tyr Gln Ile Lys Asp Gly
1445 1450 1455

His Val Ile Leu Gln Leu Asn Ser Ile Pro Ser Ser Asp Phe Leu
1460 1465 1470

Cys Val Arg Phe Arg Ile Phe Glu Leu Phe Glu Val Gly Phe Leu
1475 1480 1485

Ser Pro Ala Thr Phe Thr Val Tyr Glu Tyr His Arg Pro Asp Lys
1490 1495 1500

Gln Cys Thr Met Phe Tyr Ser Thr Ser Asn Ile Lys Ile Gln Lys
1505 1510 1515

Val Cys Glu Gly Ala Ala Cys Lys Cys Val Glu Ala Asp Cys Gly
1520 1525 1530

Gln Met Gln Glu Glu Leu Asp Leu Thr Ile Ser Ala Glu Thr Arg
1535 1540 1545

Lys Gln Thr Ala Cys Lys Pro Glu Ile Ala Tyr Ala Tyr Lys Val
1550 1555 1560

Ser Ile Thr Ser Ile Thr Val Glu Asn Val Phe Val Lys Tyr Lys
1565 1570 1575

Ala Thr Leu Leu Asp Ile Tyr Lys Thr Gly Glu Ala Val Ala Glu
1580 1585 1590

Lys Asp Ser Glu Ile Thr Phe Ile Lys Lys Val Thr Cys Thr Asn
1595 1600 1605

Ala Glu Leu Val Lys Gly Arg Gln Tyr Leu Ile Met Gly Lys Glu

```
            1610                1615                1620

        Ala Leu  Gln Ile Lys Tyr Asn  Phe Ser Phe Arg Tyr  Ile Tyr Pro
            1625                1630                1635

        Leu Asp  Ser Leu Thr Trp Ile  Glu Tyr Trp Pro Arg  Asp Thr Thr
            1640                1645                1650

        Cys Ser  Ser Cys Gln Ala Phe  Leu Ala Asn Leu Asp  Glu Phe Ala
            1655                1660                1665

        Glu Asp  Ile Phe Leu Asn Gly  Cys
            1670                1675
```

**Description**

Cross-Reference to Related Applications

This application claims priority to and the benefit of U.S. Provisional Patent Application Serial No. 61/806,687, filed March 29, 2013, the entire content of which is hereby incorporated by reference.

Technical Field

The field of the invention is medicine, immunology, molecular biology, and protein chemistry.

Background

The complement system acts in conjunction with other immunological systems of the body to defend against intrusion of cellular and viral pathogens. There are at least 25 complement proteins, which are found as a complex collection of plasma proteins and membrane cofactors. The plasma proteins make up about 10% of the globulins in vertebrate serum. Complement components achieve their immune defensive functions by interacting in a series of intricate but precise enzymatic cleavage and membrane binding events. The resulting complement cascade leads to the production of products with opsonic, immunoregulatory, and lytic functions. A concise summary of the biologic activities associated with complement activation is provided, for example, in The Merck Manual, 16th Edition.

The complement cascade can progress via the classical pathway (CP), the lectin pathway, or the alternative pathway (AP). The lectin pathway is typically initiated with binding of mannose-binding lectin (MBL) to high mannose substrates. The AP can be antibody independent, and can be initiated by certain molecules on pathogen surfaces. The CP is typically initiated by antibody recognition of, and binding to, an antigenic site on a target cell. These pathways converge at the C3 convertase - the point where complement component C3 is cleaved by an active protease to yield C3a and C3b.

The AP C3 convertase is initiated by the spontaneous hydrolysis of complement component C3, which is abundant in the plasma fraction of blood. This process, also known as "tickover," occurs through the spontaneous cleavage of a thioester bond in C3 to form C3i or C3($H_2O$). Tickover is facilitated by the presence of surfaces that support the binding of activated C3 and/or have neutral or positive charge characteristics (e.g., bacterial cell surfaces). This formation of C3($H_2O$) allows for the binding of plasma protein Factor B, which in turn allows Factor D to cleave Factor B into Ba and Bb. The Bb fragment remains bound to C3 to form a complex containing C3($H_2O$)Bb - the "fluid-phase" or "initiation" C3 convertase. Although only produced in small amounts, the fluid-phase C3 convertase can cleave multiple C3 proteins into C3a and C3b and results in the generation of C3b and its subsequent covalent binding to a surface (e.g., a bacterial surface). Factor B bound to the surface-bound C3b is cleaved by Factor D to thus form the surface-bound AP C3 convertase complex containing C3b,Bb. (See, e.g., Müller-Eberhard (1988) Ann Rev Biochem 57:321-347.)

The AP C5 convertase - $(C3b)_2$,Bb - is formed upon addition of a second C3b monomer to the AP C3 convertase. (See, e.g., Medicus et al. (1976) J Exp Med 144:1076-1093 and Fearon et al. (1975) J Exp Med 142:856-863.) The role of the second C3b molecule is to bind C5 and present it for cleavage by Bb. (See, e.g., Isenman et al. (1980) J Immunol 124:326-331.) The AP C3 and C5 convertases are stabilized by the addition of the trimeric protein properdin as described in, e.g., Medicus et al. (1976), *supra.* However, properdin binding is not required to form a functioning alternative pathway C3 or C5 convertase. (See, e.g., Schreiber et al. (1978) Proc Natl Acad Sci USA 75: 3948-3952 and Sissons et al. (1980) Proc Natl Acad Sci USA 77: 559-562.)

The CP C3 convertase is formed upon interaction of complement component C1, which is a complex of C1q, C1r, and C1s, with an antibody that is bound to a target antigen (e.g., a microbial antigen). The binding of the C1q portion of C1 to the antibody-antigen complex causes a conformational change in C1 that activates Clr. Active C1r then cleaves the C1-associated C1s to thereby generate an active serine protease. Active C1s cleaves complement component C4 into C4b and C4a. Like C3b, the newly generated C4b fragment contains a highly reactive thiol that readily forms amide or ester bonds with suitable molecules on a target surface (e.g., a microbial cell surface). C1s also cleaves complement component C2 into C2b and C2a. The complex formed by C4b and C2a is the CP C3 convertase, which is capable of processing C3 into C3a and C3b. The CP C5 convertase - C4b,C2a,C3b - is formed upon addition of a C3b monomer to the CP C3 convertase. (See, e.g., Müller-Eberhard (1988), *supra* and Cooper et al. (1970) J Exp Med 132:775-793.)

In addition to its role in C3 and C5 convertases, C3b also functions as an opsonin through its interaction with complement receptors present on the surfaces of antigen-presenting cells such as macrophages and dendritic cells. The opsonic function of C3b is generally considered to be one of the most important anti-infective functions of the complement system. Patients with genetic lesions that block C3b function are prone to infection by a broad variety of pathogenic organisms, while patients with lesions later in the complement cascade sequence, i.e., patients with lesions that block C5 functions, are found to be more prone only to *Neisseria* infection, and then only somewhat more prone.

The AP and CP C5 convertases cleave C5 into C5a and C5b. Cleavage of C5 releases C5a, a potent anaphylatoxin and chemotactic factor, and C5b, which allows for the formation of the lytic terminal complement complex, C5b-9. C5b combines with C6, C7, and C8 to form the C5b-8 complex at the surface of the target cell. Upon binding of several C9 molecules, the membrane attack complex (MAC, C5b-9, terminal complement complex - TCC) is formed. When sufficient numbers of MACs insert into target cell membranes the openings they create (MAC pores) mediate rapid osmotic lysis of the target cells.

While a properly functioning complement system provides a robust defense against infecting microbes, inappropriate regulation or activation of the complement pathways has been implicated in the pathogenesis of a variety of disorders including, e.g., rheumatoid arthritis (RA); lupus nephritis; asthma; ischemia-reperfusion injury; atypical hemolytic uremic syndrome (aHUS); dense deposit disease (DDD); paroxysmal nocturnal hemoglobinuria (PNH); macular degeneration (e.g., age-related macular degeneration (AMD)); hemolysis, elevated liver enzymes, and low platelets (HELLP) syndrome; thrombotic thrombocytopenic purpura (TTP); spontaneous fetal loss; Pauci-immune vasculitis; epidermolysis bullosa; recurrent fetal loss; multiple sclerosis (MS); traumatic brain injury; and injury resulting from myocardial infarction, cardiopulmonary bypass and hemodialysis. (See, e.g., Holers et al. (2008) Immunological Reviews 223:300-316.) The down-regulation of complement activation has been demonstrated to be effective in treating several disease indications in a variety of animal models. See, e.g., Rother t al. (2007) Nature Biotechnology 25(11):1256-1264; Wang et al. (1996) Proc. Natl. Acad. Sci. USA 93:8563-8568; Wang et al. (1995) Proc. Natl. Acad. Sci. USA 92:8955-8959; Rinder et al. (1995) J. Clin. Invest. 96:1564-1572; Kroshus et al. (1995) Transplantation 60:1194-1202; Homeister et al. (1993) J. Immunol. 150:1055-1064; Weisman et al. (1990) Science 249:146-151; Amsterdam et al. (1995) Am. J. Physiol. 268:H448-H457; and Rabinovici et al. (1992) J Immunol 149:1744 1750.

## Summary

The present disclosure relates to compositions and methods for prolonging the half-life of a therapeutic agent - e.g., a C5 antagonist such as an anti-C5 antibody - in the serum of a subject (e.g., a human). The inventors appreciated that antigen-mediated clearance (i.e., C5-driven clearance) contributes significantly to reducing the serum half-life of an antagonist anti-C5 antibody, such as eculizumab. While anti-C5 antibodies are highly effective at inhibiting complement *in vitro* and *in vivo* (see, e.g., Hillmen et al. (2004) N Engl J Med 350(6):552), the antibodies are particularly susceptible to target-mediated clearance because of the high concentration of C5 in blood (see International application publication no. WO 2010/151526). The concentration of C5 protein in human serum is approximately 75 μg/mL (0.4 μM) (Rawal and Pangburn (2001) J Immunol 166(4):2635-2642) and the protein is rapidly turned over. In fact, the half-life of human C5 in blood is approximately 63 hours. See Sissons et al. (1977) Clin Invest 59:704-715. The abundance of C5 in serum requires a correspondingly high concentration of a C5 antagonist (e.g., an anti-C5 antibody such as eculizumab) to effectively inhibit C5 in humans, e.g., humans afflicted with a complement-associated disorder such as paroxysmal nocturnal hemoglobinuria (PNH). Yet, because a C5-bound anti-C5 antibody is eliminated at roughly the same rate as C5, as compared to the slower clearance rate of an antibody expected in the absence of antigen-mediated clearance, sustained inhibition of C5 and complement activity in patients in need thereof also requires higher frequency administration of the antibody.

The inventors further appreciated that reducing the concentration of C5 in the serum, and particularly administering a C5 antagonist to a human in whom the concentration of C5 is reduced, provides at least two advantages: (a) reducing the required dosage amount of the C5 antagonist and/or (b) reducing the required dosage frequency of the C5 antagonist. A C5 antagonist is generally administered to patients afflicted with hemolytic disease in an amount necessary to maintain serum complement activity below 20% of the complement activity in normal serum in the absence of the C5 antagonist. For eculizumab, the concentration of the antibody required to maintain serum complement activity below this level is approximately greater than or equal to 35 to 50 μg/mL (i.e., approximately 1 mole of eculizumab per 2 moles of C5). See, e.g., International patent application publication nos. WO 2005/074607 and WO 2010/054403. Below the 20% threshold, complement activity is sufficiently reduced to control, among other things, C5b-mediated hemolysis in the patients. However, generally, if the complement activity in such a patient exceeds that 20% threshold, the patient will experience a breakthrough event. For a patient suffering from PNH, this breakthrough event is characterized by hemoglobinuria, dysphagia, and increased risk for thrombosis. See, e.g., International patent application publication no. WO 2005/074607. For a patient suffering from atypical hemolytic uremic syndrome (aHUS), breakthrough can be even more devastating - thrombosis and kidney failure. See International patent application publication no. WO 2010/054403.

Reducing the concentration of C5 in the serum effectively reduces the concentration of the C5 antagonist required to maintain serum complement activity at below 20%. Thus, when the serum concentration of C5 is reduced, the dose amount of a C5 antagonist (e.g., an anti-C5 antibody such as eculizumab) can be less than the dose amount of the antagonist required for maintaining serum complement activity at below 20% when the serum C5 concentration is not reduced.

In addition or in the alternative, a lower level of C5 in the serum can significantly reduce the impact of C5 antigen-mediated clearance on an antagonist anti-C5 antibody administered to a human. Since the synthetic rate of C5 drives the rate at which a C5 antagonist such as eculizumab is cleared, a reduction in that synthetic rate by virtue of, e.g., an siRNA that inhibits expression of C5, would correspondingly prolong serum residency of the C5 antagonist. Thus, a medical practitioner treating a patient afflicted with a complement-associated disorder can administer a C5 antagonist to the patient less frequently and/or at a lower dose and still achieve clinical efficacy for an equal or longer period of time. The ability to administer a lower dose of the C5 antagonist, or the ability to administer the C5 antagonist less frequently, also allows for additional delivery routes such as, e.g., subcutaneous administration or intramuscular administration and opportunities for self-administration by patients in their homes.

Accordingly, the disclosure provides a method for increasing the half-life of a therapeutic agent in the serum of a subject (e.g., a human). The method comprises: (i) administering to the subject a compound that reduces in the subject the serum concentration of the antigen (e.g., soluble antigen) to which a therapeutic agent binds to thereby reduce the concentration of the antigen in the serum of the human and (ii) administering to the subject the therapeutic agent, wherein a reduced antigen concentration in the serum of the subject increases the serum half-life of the therapeutic agent administered to the subject. The therapeutic agent can be, e.g., an antibody or antigen-binding fragment thereof, a small molecule, an aptamer, or a non-antibody, scaffold protein.

In some embodiments, antigen to which the therapeutic agent binds is a complement protein (e.g., a human protein) such as, e.g., C1, C4, C3, C2, C5, C6, C7, C8, C9, properdin, complement factor B, complement factor D, MBL, MASP1, MASP2, or MASP3.

Also featured is a method for increasing the half-life of a C5 antagonist (e.g., an anti-C5 antibody) in the serum of a human, which method includes: (i) administering to the human a compound that reduces the concentration of complement component C5 in the serum of the human to thereby reduce the C5 concentration in the serum of the human and (ii) administering to the human the C5 antagonist, wherein a reduced C5 concentration in the serum of the human increases the serum half-life of the C5 antagonist administered to the human.

As used herein, the term "C5 antagonist" or like terms means any agent that binds to complement component C5 protein and inhibits the cleavage of C5 into fragments C5a and C5b by C5 convertase (e.g., an alternative pathway or classical pathway C5 convertase). As used herein, the term "C5 convertase" can refer to either the classical pathway C5 convertase C4bC2aC3b or the alternative pathway convertase $(C3b)_2Bb$.

As noted above, administration of the C5 antagonist (e.g., an antagonist anti-C5 antibody) to the human in a context in which the serum concentration of C5 is reduced below normal levels has several advantageous effects, e.g., for humans having, suspected of having, or at risk for developing, a complement-associated condition. For example, because there is less C5 antigen in the serum of the human, the amount of the C5 antagonist administered to the human having the reduced C5 concentration can be less than the therapeutically effective amount of the C5 antagonist required without the reduction in C5 concentration (that is in the context of normal serum C5 concentration). In addition, or in the alternative, the frequency of administration of a therapeutically effective amount of the C5 antagonist to the human having a reduced C5 concentration can be less often than the frequency of administration of the therapeutically effective amount to the human required without the reduction in C5 concentration. For example, instead of once, biweekly dosing, the required dosing frequency could be extended to, e.g., once monthly, once bimonthly, or even once every three months. Thus, reducing the serum C5 concentration can one or both of: (a) reduce the amount of the dose of the C5 antagonist required and (b) the frequency of dosing of the C5 antagonist, and yet still achieve the same therapeutic effect in the human as a higher dose and/or more frequent administration of the C5 antagonist required if the human had a normal serum C5 concentration.

Thus, in another aspect, the disclosure features a method for decreasing the frequency by which a therapeutically effective amount of a C5 antagonist must be administered to a human having, suspected of having, or at risk for developing, a complement-associated disorder. The method includes: (i) administering to the human a compound that reduces the concentration of complement component C5 in the serum of the human to thereby reduce the C5 concentration in the serum of the human and (ii) administering to the human a therapeutically effective amount of the C5 antagonist, wherein a reduced C5 concentration in the serum of the human decreases the frequency by which a therapeutically effective amount of a C5 antagonist must be administered to the human.

Moreover, in another aspect, the disclosure features a method for decreasing the dosage of a C5 antagonist required for therapeutic efficacy in a human having, suspected of having, or at risk for developing, a complement-associated disorder. The method comprises: (i) administering to the human a compound that reduces the concentration of complement component C5 in the serum of the human to thereby reduce the C5 concentration in the serum of the human and (ii) administering to the human a therapeutically effective amount of the C5 antagonist, wherein a reduced C5 concentration in the serum of the human decreases the dosage of a C5 antagonist required for therapeutic efficacy in the human.

Also featured is a method for treating a subject (e.g., a human) afflicted with a complement-associated disorder. The method comprises administering to the subject a therapeutically-effective amount of an antagonist of complement component C5, wherein the subject has a reduced serum concentration of C5, relative to the normal serum concentration of C5, as the result of the prior administration to the patient of a compound that reduces the serum concentration of C5. In some embodiments, the normal serum concentration of C5 is the average C5 concentration of like healthy subjects of the same species. In some embodiments, the normal serum concentration of C5 is the serum C5 concentration in the subject (e.g., human) prior to administration of the compound and subsequent reduction of C5 concentration.

The disclosure also provides a method for treating a subject afflicted with a complement-associated disorder, which method comprises administering to the subject a compound that reduces the serum concentration of C5 in the subject in an amount effective to reduce the serum concentration of C5 in the subject. The compound is also to be used therapeutically in conjunction with an inhibitor of C5, wherein, as a result of administration of the compound, the inhibitor of C5 can be administered to the subject (e.g., a human) less frequently and/or in lower dose amounts than the frequency of administration and/or dose amount that would be administered if the subject had a normal serum concentration of C5. Thus, the subject is one who is to be treated with a C5 inhibitor, and/or in need of treatment with the C5 inhibitor, as part of the therapeutic strategy for treating the subject's complement-associated disorder.

Further disclosed is a method for treating a subject (e.g., a human) afflicted with a complement-associated disorder, which method comprises administering to the subject a compound that reduces the serum concentration of complement component C5, wherein the subject is also to be treated with a C5 antagonist.

In another aspect, the disclosure provides a composition (e.g., a pharmaceutical composition) comprising a compound that reduces the serum concentration of C5 in a subject (e.g., a human), the composition for use in treating a subject afflicted with a complement-associated condition. The subject can be, e.g., one who is to be administered a C5 antagonist (e.g., an anti-C5 antibody).

In another aspect, the disclosure features a composition (e.g., a pharmaceutical composition) comprising a C5 antagonist (e.g., an anti-C5 antibody) for use in treating a subject afflicted with a complement-associated disorder. The subject can be one who has a reduced level of C5 expression as a result of the action of a compound (that reduces expression of C5) administered to the subject.

In some embodiments of any of the methods described herein, the compound reduces the level of expression by one or more cells in the subject (e.g., human) of the antigen to which the therapeutic agent binds. Thus, in some embodiments of any of the methods described herein, the compound reduces the level of expression of human complement component C5 by one or more cells in the human.

In some embodiments of any of the methods described herein, the compound inhibits transcription of a gene encoding the antigen (e.g., a human complement component C5 gene) to which the therapeutic agent (e.g., a C5 antagonist such as an anti-C5 antibody) binds. In some embodiments of any of the methods described herein, the compound inhibits translation of an mRNA encoding the antigen to which the therapeutic agent binds. In some embodiments of any of the methods described herein, the compound reduces the stability of an mRNA encoding the antigen to which the therapeutic agent binds. Thus, in some embodiments, e.g., in embodiments where the therapeutic agent is a C5 antagonist such as an anti-C5 antibody, the compound inhibits transcription of a human complement component C5 gene. In some embodiments, the compound inhibits translation of an mRNA encoding human complement component C5. In some embodiments, the compound reduces the stability of an mRNA encoding human complement component C5. In some embodiments, the compound is an siRNA specific for the mRNA encoding the antigen to which the therapeutic agent binds (e.g., an siRNA specific for human complement component C5). In some embodiments, the compound is an antisense nucleic acid complementary to a mRNA encoding the antigen to which the therapeutic agent binds (e.g., an antisense nucleic acid complementary to a mRNA encoding human complement component C5).

In some embodiments of any of the methods described herein, the C5 antagonist is selected from the group consisting of: MB12/22, MB12/22-RGD, ARC187, ARC1905, SSL7, and OmCI.

In some embodiments of any of the methods described herein, the C5 antagonist is an antibody, or an antigen-binding fragment thereof, that binds to complement component C5 and inhibits the cleavage of C5 by the C5 convertase into fragments C5a and C5b. The antibody or antigen-binding fragment thereof can be, e.g., a polyclonal antibody, a recombinant antibody, a diabody, a chimerized or chimeric antibody, a deimmunized antibody, a fully human antibody, a single chain antibody, a domain antibody, an Fv fragment, an Fd fragment, an Fab fragment, an Fab' fragment, or an $F(ab')_2$ fragment. In some embodiments, the antibody or antigen-binding fragment thereof is a bispecific antibody or bispecific antigen-binding fragment. In some embodiments, the antibody or antigen-binding fragment thereof can be a DVD-Ig antibody.

In some embodiments of any of the methods described herein, the antibody is eculizumab. In some embodiments of any of the methods described herein, the antigen-binding fragment of an anti-C5 antibody is pexelizumab.

In some embodiments of any of the methods described herein, the compound reduces the serum concentration of the antigen (e.g., human C5) to which the therapeutic agent binds by at least 2 (e.g., at least 3, 4, 5, 6, 7, 8, 9, 10, 15, 20, 25, 30, 35, 40, 45, 50, 55, 60, 65, 70, 75, 80, 85, 90, 95, or 100) %. For example, in some embodiments of any of the methods described herein, the compound reduces the serum concentration of C5 by at least 5%, 10%, 20%, 30%, 40%, 50%, 60%, or 70%.

In some embodiments, the serum concentration of C5 is reduced to 40% or less (e.g., 39%, 38%, 37%, 36%,

35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%) of the normal serum concentration of C5 (e.g., the average serum concentration of C5 among individuals or the serum C5 concentration in the serum of the patient prior to treatment with the compound).

In some embodiments, the compound is administered to the patient in an amount effective to reduce the expression of C5 protein and/or mRNA by liver cells by at least 20 (e.g., at least 25, 30, 35, 40, 45, 50, 55, 60, 65, 70, 75, 80, 85, 90, or 95) %.

In some embodiments of any of the methods described herein, the compound can be chronically administered to the subject (e.g., the human). In some embodiments of any of the methods described herein, the therapeutic agent (e.g., a C5 antagonist) can be chronically administered to the subject (e.g., the human). In some embodiments of any of the methods described herein, both the compound (e.g., a nucleic acid compound that reduces expression of C5) and the therapeutic agent (e.g., a C5 antagonist) can be chronically administered to the subject (e.g., the human). As used herein, "chronically administered," "chronic treatment," "treating chronically," or similar grammatical variations thereof refer to a treatment regimen that is employed to maintain a certain threshold concentration of a compound or therapeutic agent in the blood, serum, or plasma of a patient required for activity in the patient over a prolonged period of time. Accordingly, a patient chronically treated with a compound can be treated for a period of time that is greater than or equal to 2 weeks (e.g., 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks; 1, 2, 3, 4, 5, 6, 7, 8, 9, 10, 11, or 12 months; or 1, 1.5, 2, 2.5, 3, 3.5, 4, 4.5, 5, 5.5, 6, 6.5, 7, 7.5, 8, 8.5, 9, 9.5, 10, 10.5, or 12 years or for the remainder of the patient's life) with the compound in an amount and with a dosing frequency that are sufficient to maintain in the patient's blood a reduction in the concentration of the antigen to which the therapeutic agent binds. Similarly, a patient chronically treated with a C5 antagonist such as an anti-C5 antibody can be treated for a period of time that is greater than or equal to 2 weeks (e.g., 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks; 1, 2, 3, 4, 5, 6, 7, 8, 9, 10, 11, or 12 months; or 1, 1.5, 2, 2.5, 3, 3.5, 4, 4.5, 5, 5.5, 6, 6.5, 7, 7.5, 8, 8.5, 9, 9.5, 10, 10.5, or 12 years or for the remainder of the patient's life) with the therapeutic agent in an amount and with a dosing frequency that are sufficient to maintain inhibition or substantial inhibition (less than 20% of the complement activity present in a human in the absence of a C5 antagonist) of systemic complement activity in the patient. In some embodiments, the complement inhibitor can be chronically administered to a patient in need thereof in an amount and with a frequency that are effective to maintain serum hemolytic activity at less than or equal to 20 (e.g., 19, 18, 17, 16, 15, 14, 13, 12, 11, 10, 9, 8, 7, 6, or even below 5) %. *See,* e.g., Hill et al. (2005) Blood 106(7):2559. In some embodiments, the complement inhibitor can be administered to a patient in an amount and with a frequency that are effective to maintain serum lactate dehydrogenase (LDH) levels at within at least 20 (e.g., 19, 18, 17, 16, 15, 14, 13, 12, 11, 10, 9, 8, 7, 6, or even below 5) % of the normal range for LDH. *See* Hill et al. (2005) *supra.* In some embodiments, the complement inhibitor is administered to the patient in an amount and with a frequency that are effective to maintain a serum LDH level less than 550 (e.g., less than 540, 530, 520, 510, 500, 490, 480, 470, 460, 450, 440, 430, 420, 410, 400, 390, 380, 370, 360, 350, 340, 330, 320, 310, 300, 290, 280, or less than 270) IU/L. As noted above, administering a C5 antagonist (e.g., an anti-C5 antibody such as eculizumab) in the context of reduced serum C5 levels can reduce the amount (dose) of the C5 antagonist required for therapeutic efficacy and/or can reduce how often the same dose or a reduced dose must be administered to the patient, and still maintain therapeutic complement inhibition.

In some embodiments of any of the methods described herein, the compound is administered to the human at least once weekly for at least three weeks. In some embodiments of any of the methods described herein, the compound is administered to the human at least once weekly for at least six weeks. In some embodiments of any of the methods described herein, the compound is administered to the human at least once weekly for at least six months.

In some embodiments of any of the methods described herein, the compound is subcutaneously administered to the human. In some embodiments of any of the methods described herein, the therapeutic agent (e.g., a C5 antagonist) and the compound are administered to the human using different routes of administration. In some embodiments of any of the methods described herein, the therapeutic agent (e.g., a C5 antagonist) and the compound are administered to the human under different dosing schedules.

In some embodiments, each dose of the compound administered to the patient is between 0.01 mg to 30 mg per kg weight of the patient. In some embodiments, the dose is between 0.01 mg/kg and 35 mg/kg (e.g., 0.1 mg/kg - 10 mg/kg, 1 mg/kg - 15 mg/kg, 0.1 mg/kg - 5 mg/kg, 3 mg/kg - 5 mg/kg, 10 mg/kg - 30 mg/kg, 15 mg/kg - 35 mg/kg, 1 mg/kg - 3 mg/kg, 10 mg/kg - 20 mg/kg, 0.01 mg/kg - 1 mg/kg, 5 mg/kg - 20 mg/kg, 5 mg/kg - 15 mg/kg, or 2 mg/kg to 15 mg/kg). In some embodiments, the dose of the compound is at least 0.01 (e.g., 0.1, 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36) mg/kg. In some embodiments the dose of the compound is at least 0.01 mg/kg (e.g., at least 0.1, 0.5, 1, 2, 3, 4, 5, 6, 7, 8, 9, or 10) mg/kg, but no greater than 50 (e.g., 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, or 11) mg/kg.

In some embodiments, the serum half-life of the therapeutic agent (e.g., the C5 antagonist such as an anti-C5 antibody) is increased by at least 2 (e.g., at least 3, 4, 5, 6, 7, 8, 9, 10, 20, 50, or 100) fold in the context of a reduced serum concentration of the antigen to which the therapeutic agent binds. That is, in some embodiments of any of the methods described herein, the serum half-life of a C5 antagonist such as an anti-C5 antibody is increased by at least 2 (e.g., at least 3, 4, 5, 6, 7, 8, 9, 10, 20, 50, or 100) fold in the context of a reduced serum concentration of C5. For example, in some embodiments of any of the methods described herein, the serum half-life of a therapeutic agent can be increased from, e.g., 3 days to 14 days, from 2.5 days to 10 days, from 2 days to 15 days, from 5 days to 15 days, from 10 days to 20 days, and so on.

As noted above, in some embodiments of any of the methods described herein, the therapeutically effective amount of the C5 antagonist administered to the human having a reduced C5 concentration is less than the therapeutically effective amount of the C5 antagonist required without the reduction in C5 concentration. The terms "therapeutically effective amount" or "therapeutically effective dose," or similar terms used herein are intended to mean an amount of an agent (e.g., a therapeutic agent such as a C5 antagonist) that will elicit the desired biological or medical response (e.g., an improvement in one or more symptoms of a complement-associated disorder and/or inhibition of complement activity).

In some embodiments of any of the methods described herein, the frequency of administration of the C5 antagonist to the human having a reduced C5 concentration is less often than the frequency of administration of the C5 antagonist to the human required without the reduction in C5 concentration. For example, in embodiments in which the C5 antagonist is an anti-C5 antibody, the frequency of administration of the C5 antagonist to the human having a reduced C5 concentration is no more frequently than once monthly. In another example, in embodiments in which the C5 antagonist is an anti-C5 antibody, the frequency of administration of the C5 antagonist to the human having a reduced C5 concentration is no more frequently than once every two months.

In some embodiments of any of the methods described herein, the complement-associated disorder is selected from the group consisting of paroxysmal nocturnal hemoglobinuria (PNH), atypical hemolytic-uremic syndrome (aHUS), shiga toxin *E. coli*-related hemolytic uremic syndrome (STEC-HUS), dense deposit disease (DDD), C3 nephropathy, myasthenia gravis, neuromyelitis optica, cold agglutinin disease (CAD), antineutrophil cytoplasm antibody (ANCA)-associated vasculitis (AAV), asthma, age-related macular degeneration (AMD), transplant rejection, Goodpasture's syndrome, glomerulonephritis, vasculitis, rheumatoid arthritis, dermatitis, systemic lupus erythematosus (SLE), Guillain-Barré syndrome (GBS), dermatomyositis, psoriasis, Graves' disease, Hashimoto's thyroiditis, type I diabetes, pemphigus, autoimmune hemolytic anemia (AIHA), idiopathic thrombocytopenic purpura (ITP), lupus nephritis, ischemia-reperfusion injury, thrombotic thrombocytopenic purpura (TTP), Pauci-immune vasculitis, epidermolysis bullosa, multiple sclerosis, spontaneous fetal loss, recurrent fetal loss, traumatic brain injury, injury resulting from myocardial infarction, cardiopulmonary bypass and hemodialysis, and hemolysis, elevated liver enzymes, and low platelets (HELLP) syndrome.

"Polypeptide," "peptide," and "protein" are used interchangeably and mean any peptide-linked chain of amino acids, regardless of length or post-translational modification. As noted below, the polypeptides described herein can be, e.g., wild-type proteins, functional fragments of the wild-type proteins, or variants of the wild-type proteins or fragments. Variants, in accordance with the disclosure, can contain amino acid substitutions, deletions, or insertions. The substitutions can be conservative or non-conservative. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

As used herein, percent (%) amino acid sequence identity is defined as the percentage of amino acids in a candidate sequence that are identical to the amino acids in a reference sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST software. Appropriate parameters for measuring alignment, including any algorithms needed to achieve maximal alignment over the full-length of the sequences being compared can be determined by known methods.

Unless otherwise defined, all technical and scientific terms used herein have the same meaning as commonly understood by one of ordinary skill in the art to which this disclosure pertains. In case of conflict, the present document, including definitions, will control. Preferred methods and materials are described below, although methods and materials similar or equivalent to those described herein can also be used in the practice or testing of the presently disclosed methods and compositions. All publications, patent applications, patents, and other references mentioned herein are incorporated by reference in their entirety.

Other features and advantages of the present disclosure, e.g., methods for increasing the half-life of a therapeutic agent (e.g., a C5 antagonist), will be apparent from the following description, the examples, and from the claims.

Brief Description of the Drawings

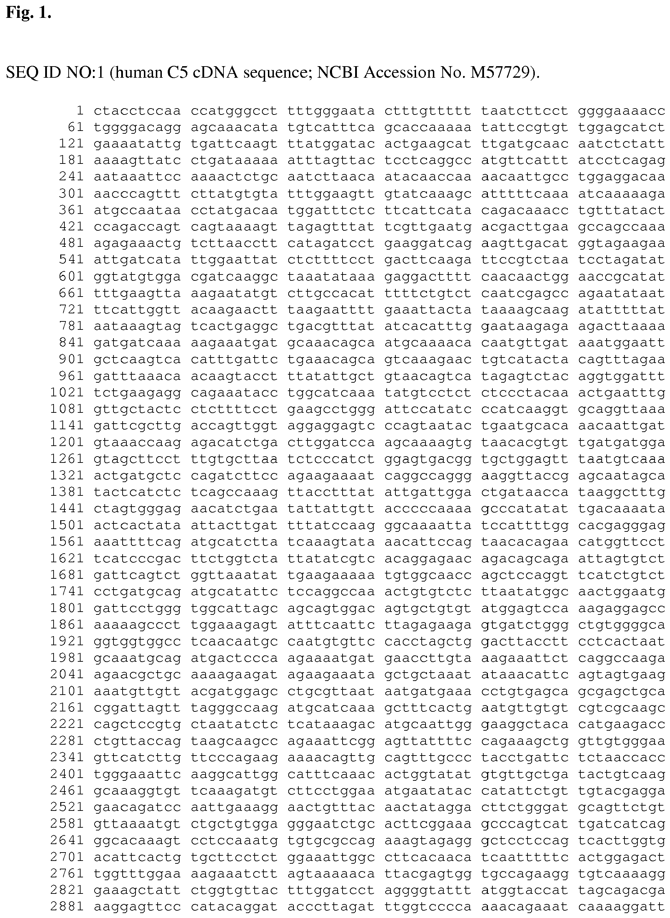

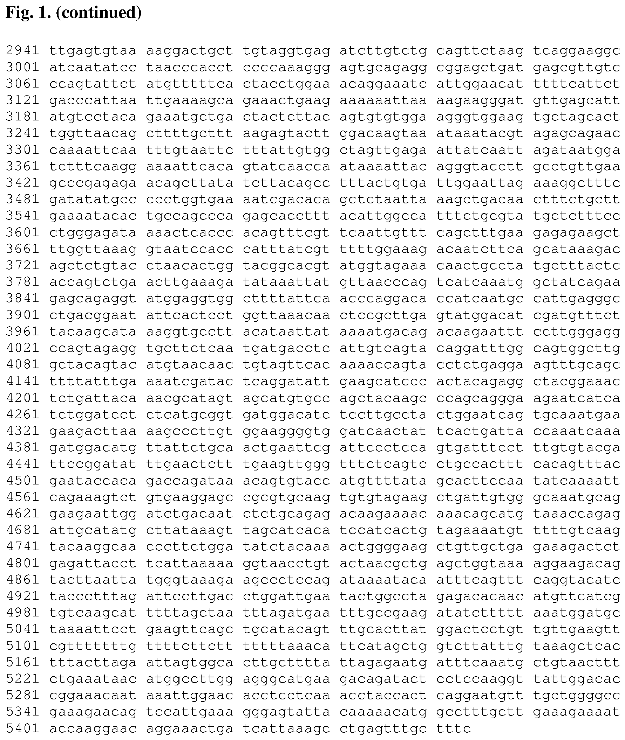

**Fig. 1** depicts an exemplary nucleotide sequence for human complement component C5 (SEQ ID NO:1).

**Fig. 2** depicts an exemplary amino acid sequence for human complement component pro-C5 (SEQ ID NO:2).

Detailed Description

The disclosure features compositions (e.g., compounds and therapeutic agents such as C5 antagonists) and methods for prolonging the half-life of a therapeutic agent in the serum of a human. For example, the disclosure provides methods for prolonging the serum half-life of a C5 antagonist (e.g., an anti-C5 antibody) in a human by reducing the concentration of C5 in the serum of the human. While in no way meant to be limiting, exemplary compositions, conjugates, pharmaceutical compositions and formulations, methods for generating such compositions, as well as methods for using the compositions are set forth below.

Compounds that Reduce Serum Concentration of Complement Component C5

As used herein, "a compound that reduces the concentration of complement component C5 in serum" is any agent that inhibits: (i) the expression of a complement component C5 protein; (ii) the proper intracellular trafficking, the post-translational modification, or secretion by a cell, of a complement component C5 protein; or (iii) the stability of a C5 protein in the serum of a subject, which ultimately has the impact of reducing the concentration of C5 in the serum of a subject (e.g., a human). Inhibition of complement component C5 protein expression includes: inhibition of transcription of a gene encoding a human C5 protein; increased degradation of an mRNA encoding a human C5 protein; and/or inhibition of translation of an mRNA encoding a human C5 protein. Accordingly, a compound can reduce serum C5 concentration through, among other things, increased degradation of a human C5 protein; inhibition of proper processing of a pre-pro human C5 protein; or inhibition of proper trafficking or secretion by a cell of a human C5 protein. In some embodiments, the compound does not include an antibody that binds to C5. The compound can be, e.g., a small molecule, a nucleic acid (e.g., an siRNA or an antisense oligonucleotide), a protein, or an aptamer.

In some embodiments, the compound is one that inhibits expression of C5 protein. Nucleic acid inhibitors, e.g., can be used to decrease expression of an endogenous gene encoding human complement component C5. The nucleic acid antagonist can be, e.g., an siRNA, a dsRNA, a ribozyme, a triple-helix former, an aptamer, or an antisense nucleic acid. siRNAs are small double stranded RNAs (dsRNAs) that optionally include overhangs. For example, the duplex region of an siRNA can be about 18 to 25 nucleotides in length, e.g., about 19, 20, 21, 22, 23, or 24 nucleotides in length. The siRNA sequences can be, in some embodiments, exactly complementary to the target mRNA. dsRNAs and siRNAs in particular can be used to silence gene expression in mammalian cells (e.g., human cells). One of skill in the art would understand how to design such inhibitory nucleic acids in view of the human C5 coding sequence (e.g., SEQ ID NO:1). See, e.g., Clemens et al. (2000) Proc. Natl. Acad. Sci. USA 97:6499- 6503; Billy et al. (2001) Proc. Natl. Acad. Sci. USA 98:14428-14433; Elbashir et al. (2001) Nature 411:494-8; Yang et al. (2002) Proc. Natl. Acad. Sci. USA 99:9942-9947, and U.S. patent application publication nos. 20030166282, 20030143204, 20040038278, and 20030224432. Anti-sense agents can include, for example, from about 8 to about 80 nucleobases (i.e. from about 8 to about 80 nucleotides), e.g., about 8 to about 50 nucleobases, or about 12 to about 30 nucleobases. Anti-sense compounds include ribozymes, external guide sequence (EGS) oligonucleotides (oligozymes), and other short catalytic RNAs or catalytic oligonucleotides which hybridize to the target nucleic acid and modulate its expression. Anti-sense compounds can include a stretch of at least eight consecutive nucleobases that are complementary to a sequence in the target gene. An oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired, i.e., under physiological conditions in the case of *in vivo* assays or therapeutic treatment or, in the case of *in vitro* assays, under conditions in which the assays are conducted. Hybridization of antisense oligonucleotides with mRNA (e.g., an mRNA encoding a human C5 protein) can interfere with one or more of the normal functions of mRNA. The functions of mRNA to be interfered with include all key functions such as, for example, translocation of the RNA to the site of protein translation, translation of protein from the RNA, splicing of the RNA to yield one or more mRNA species, and catalytic activity which may be engaged in by the RNA. Binding of specific protein(s) to the RNA may also be interfered with by antisense oligonucleotide hybridization to the RNA. Exemplary antisense compounds include DNA or RNA sequences that specifically hybridize to the target nucleic acid, e.g., the mRNA encoding a human complement component C5 protein. The complementary region can extend for between about 8 to about 80 nucleobases. The compounds can include one or more modified nucleobases.

Modified nucleobases may include, e.g., 5-substituted pyrimidines such as 5-iodouracil, 5-iodocytosine, and Cs-propynyl pyrimidines such as Cs-propynylcytosine and $C_5$-propynyluracil. Other suitable modified nucleobases include, e.g., 7-substituted-8-aza-7-deazapurines and 7-substituted-7-deazapurines such as, for example, 7-iodo-7-deazapurines, 7-cyano-7-deazapurines, and 7-aminocarbonyl-7-deazapurines. Examples of these include 6-amino-7-iodo-7-deazapurines, 6-amino-7-cyano-7-deazapurines, 6-amino-7-aminocarbonyl-7-deazapurines, 2-amino-6-hydroxy-7-iodo-7-deazapurines, 2-amino-6-hydroxy-7-cyano-7-deazapurines, and 2-amino-6-hydroxy-7-aminocarbonyl-7-deazapurines. See, e.g., U.S. Patent Nos. 4,987,071; 5,116,742; and 5,093,246; "Antisense RNA and DNA," D.A. Melton, Ed., Cold Spring Harbor Laboratory, Cold Spring Harbor, N.Y. (1988); Haselhoff and Gerlach (1988) Nature 334:585-59; Helene (1991) Anticancer Drug D 6:569-84; Helene (1992) Ann. NY. Acad. Sci. 660:27-36; and Maher (1992) Bioassays 14:807-15.

Methods for determining whether a compound has inhibited the expression of an antigen (e.g., C5 protein) are well known in the art. Such methods for detecting protein expression include, without limitation: Western blot, dot blot, enzyme-linked immunosorbent assay (ELISA), "sandwich" immunoassays, immunoprecipitation assays, AlphaScreen® or AlphaLISA® assays, or mass spectrometry based methods.

The term "immunoassay" encompasses techniques including, without limitation, flow cytometry, FACS, enzyme immunoassays (EIA), such as enzyme multiplied immunoassay technique (EMIT), enzyme-linked immunosorbent assay (ELISA), IgM antibody capture ELISA (MAC ELISA) and microparticle enzyme immunoassay (MEIA), furthermore capillary electrophoresis immunoassays (CEIA), radio-immunoassays (RIA), immunoradiometric assays (IRMA), fluorescence polarization immunoassays (FPIA) and chemiluminescence assays (CL). If desired, such immunoassays can be automated. Immunoassays can also be used in conjunction with laser induced fluorescence. Liposome immunoassays, such as flow-injection liposome immunoassays and liposome immunosensors, are also suitable for use in the present invention. In addition, nephelometry assays, in which, for example, the formation of protein/antibody complexes results in increased light scatter that is converted to a peak rate signal as a function of the marker concentration, are suitable for use in the methods of the present invention. In a preferred embodiment of the present invention, the incubation products are detected by ELISA, RIA, fluoro immunoassay (FIA) or soluble particle immune assay (SPIA).

Generally the amount of C5 in a serum sample from a patient taken before administration of the compound is compared to the amount of C5 in a serum sample from the same patient following administration of the compound (e.g., 6 hours, 12 hours, 1 day, 36 hours, 2 days, 3 days, 1 week or 2 weeks after administration of the compound) to the patient. A reduced amount of C5 in the serum sample obtained after administration of the compound, as compared to the amount of C5 in an equivalent serum sample obtained from the patient prior to administration of the compound, indicates that the compound is one that reduces the concentration of C5 in the serum of the patient.

## C5 Antagonists

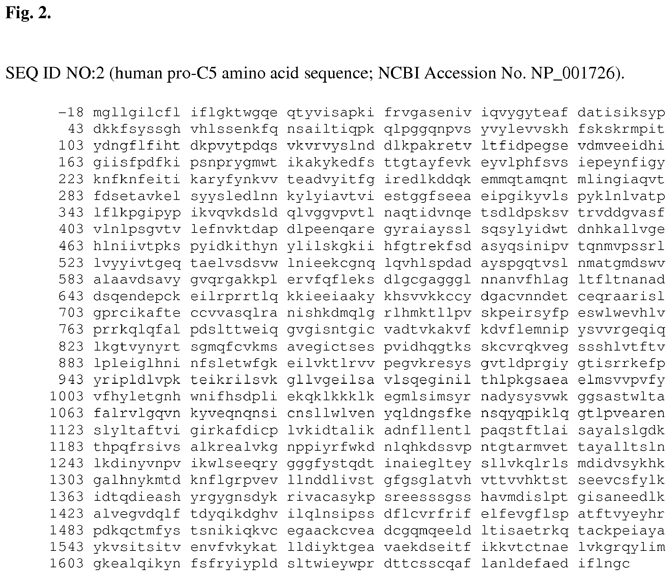

A C5 antagonist can be, e.g., a small molecule, a polypeptide, a polypeptide analog, a nucleic acid, or a nucleic acid analog. "Small molecule" as used herein, is meant to refer to an agent, which preferably has a molecular weight of less than about 6 kDa and most preferably less than about 2.5 kDa. Many pharmaceutical companies have extensive libraries of chemical and/or biological mixtures comprising arrays of small molecules, often fungal, bacterial, or algal extracts, which can be screened with any of the assays of the application. This application contemplates using, among other things, small chemical libraries, peptide libraries, or collections of natural products. Tan et al. described a library with over two million synthetic compounds that is compatible with miniaturized cell-based assays (JAm Chem Soc (1998) 120:8565-8566). It is within the scope of this application that such a library may be used to screen for inhibitors of human complement component C5. There are numerous commercially available compound libraries, such as the Chembridge DIVERSet. Libraries are also available from academic investigators, such as the Diversity set from the NCI developmental therapeutics program. Rational drug design may also be employed. For example, rational drug design can employ the use of crystal or solution structural information on the human complement component C5 protein. See, e.g., the structures described in Hagemann et al. (2008) J Biol Chem 283(12):7763-75 and Zuiderweg et al. (1989) Biochemistry 28(1):172-85. See also, Fredslund et al. (2008) Nat. Immunol. 9:753-760 and Laursen et al. (2011) EMBO J. 30:606-616. Rational drug design can also be achieved based on known compounds, e.g., a known inhibitor of C5 (e.g., an antibody, or antigen-binding fragment thereof, that binds to a human complement component C5 protein). The amino acid sequence of human C5 is known (see, Haviland et al. (1991) J. Immunol. 146:362-368) and is shown in Figure 2 as SEQ ID NO:2. Human C5 is synthesized as a pro-C5 molecule including an 18 amino acid signal peptide plus a 1658 amino acid protein that gets processed by cleavage between amino acids 655-656 and 659-660. Once fully processed, the processing results in an 18 amino acid peptide corresponding to the signal peptide, a 655 amino acid peptide (amino acids 1-655) that is referred to as the β-chain, a 4 amino acid peptide corresponding to amino acids 656-659 which is lost, and a 999 amino acid peptide corresponding to amino acids 660-1658, this being referred to as the α-chain. The α-chain and β-chain become linked to each other via disulfide bonds. This final structure of the α- and β-chains is the complete C5 molecule. This C5 can be cleaved by a C5 convertase by a cleavage between amino acids 733-734 thereby cleaving off a 74-amino acid fragment from the α-chain (corresponding to amino acids 660-733). This 74 amino acid fragment is C5a.

Peptidomimetics can be compounds in which at least a portion of a subject polypeptide is modified, and the three dimensional structure of the peptidomimetic remains substantially the same as that of the subject polypeptide. Peptidomimetics may be analogues of a subject polypeptide of the disclosure that are, themselves, polypeptides containing one or more substitutions or other modifications within the subject polypeptide sequence. Alternatively, at least a portion of the subject polypeptide sequence may be replaced with a non-peptide structure, such that the three-dimensional structure of the subject polypeptide is substantially retained. In other words, one, two or three amino acid residues within the subject polypeptide sequence may be replaced by a non-peptide structure. In addition, other peptide portions of the subject polypeptide may, but need not, be replaced with a non-peptide structure. Peptidomimetics (both peptide and non-peptidyl analogues) may have improved properties (e.g., decreased proteolysis, increased retention or increased bioavailability). Peptidomimetics generally have improved oral availability, which makes them especially suited to treatment of disorders in a human or animal. It should be noted that peptidomimetics may or may not have similar two-dimensional chemical structures, but share common three-dimensional structural features and geometry. Each peptidomimetic may further have one or more unique additional binding elements.

Aptamers are short oligonucleotide sequences that can be used to recognize and specifically bind almost any molecule, including cell surface proteins. The systematic evolution of ligands by exponential enrichment (SELEX) process is powerful and can be used to readily identify such aptamers. Aptamers can be made for a wide range of proteins of importance for therapy and diagnostics, such as growth factors and cell surface antigens. These oligonucleotides bind their targets with similar affinities and specificities as antibodies do (see, e.g., Ulrich (2006) Handb Exp Pharmacol. 173:305-326).

In some embodiments, the inhibitor of human C5 is an antibody, or antigen-binding fragment thereof, which binds to a human complement component C5 protein. (Hereinafter, the antibody may sometimes be referred to as an "anti-C5 antibody.")

In some embodiments, the C5 antagonist comprises, and/or is, eculizumab (Soliris®; Alexion Pharmaceuticals, Inc., Cheshire, CT). See, e.g., Kaplan (2002) Curr Opin Investig Drugs 3(7): 1017-23; Hill (2005) Clin Adv Hematol Oncol 3(11):849-50; and Rother et al. (2007) Nature Biotechnology 25(11):1256-1488. In some embodiments, the C5 antagonist comprises, and/or is, pexelizumab (Alexion Pharmaceuticals, Inc., Cheshire, CT). See, e.g., Whiss (2002) Curr Opin Investig Drugs 3(6):870-7; Patel et al. (2005) Drugs Today (Barc) 41(3):165-70; and Thomas et al. (1996) Mol Immunol. 33(17-18):1389-401. In some embodiments, the anti-C5 antibody or antigen-binding fragment thereof can have the same or greater affinity for C5 as does eculizumab or pexelizumab.

In some embodiments, the anti-C5 antibody or antigen-binding fragment thereof can bind to an epitope in the alpha chain of the human complement component C5 protein. Antibodies that bind to the alpha chain of C5 are described in, for example, International patent application publication no. WO 2010/136311 and U.S. patent no. 6,355,245. In some embodiments, the anti-C5 antibody can bind to an epitope in the beta chain of the human complement component C5 protein. Antibodies that bind to the C5 beta chain are described in, e.g., Moongkarndi et al. (1982) Immunobiol 162:397; Moongkarndi et al. (1983) Immunobiol 165:323; and Mollnes et al. (1988) Scand J Immunol 28:307-312. See also International patent application publication no. WO 2010/136311.

In some embodiments, the C5 antagonist can be a non-antibody, scaffold protein. These proteins are, generally, obtained through combinatorial chemistry-based adaptation of pre-existing antigen-binding proteins. For example, the binding site of human transferrin for human transferrin receptor can be modified using combinatorial chemistry to create a diverse library of transferrin variants, some of which have acquired affinity for different antigens. Ali et al. (1999) J Biol Chem 274:24066-24073. The portion of human transferrin not involved with binding the receptor remains unchanged and serves as a scaffold, like framework regions of antibodies, to present the variant binding sites. The libraries are then screened, as an antibody library is, against a target antigen of interest to identify those variants having optimal selectivity and affinity for the target antigen. Non-antibody scaffold proteins, while similar in function to antibodies, are touted as having a number of advantages as compared to antibodies, which advantages include, among other things, enhanced solubility and tissue penetration, less costly manufacture, and ease of conjugation to other molecules of interest. Hey et al. (2005) TRENDS Biotechnol 23(10):514-522.

One of skill in the art would appreciate that the scaffold portion of the non-antibody scaffold protein can include, e.g., all or part of: the Z domain of *S. aureus* protein A, human transferrin, human tenth fibronectin type III domain, kunitz domain of a human trypsin inhibitor, human CTLA-4, an ankyrin repeat protein, a human lipocalin, human crystallin, human ubiquitin, or a trypsin inhibitor from *E. elaterium. Id.*

Other exemplary C5 antagonists include the anti-C5 minibody MB 12/22 (Mubodina®; Adienne Pharma & Biotech, Bergamo, Italy) and a variant form of the minibody fused with RGD peptide, MB12/22-RGD (Ergidina®; Adienne Pharma & Biotech, Bergamo, Italy). MB12/22 and MB12/22-RGD are derived from an anti-C5 scFv, Ts-a12/22, which is described in International patent application publication no. WO 2004/007553. MB-12/22 and MB-12/22-RGD recognize an epitope comprising the C5 convertase cleavage site located between amino acids 733 and 734 of the C5 polypeptide (SEQ ID NO:2). Other anti-C5 antibodies that variously recognize epitopes on either the alpha chain or the beta chain of the C5 molecule and inhibit complement mediated hemolytic activity, are described in International patent application publication no. WO 2010/015608. C5 binding aptamers, ARC187 and ARC1905 (commercially available from Archemix/Ophthotech Corp., Princeton, NJ), are described in U.S. patent application publication no. 20070048248. OmCI, a protein excreted by the soft tick *Ornithodoros moubata,* is a naturally occurring inhibitor of C5 activity. A recombinant variant of OmCI, rev576, has also been described (Hepburn et al. (2007) J Biol Chem 282:8292-8299 and Soltys et al. (2009) Ann Neurol 65:67-75). Another naturally occurring inhibitor of C5 activity is the *Staphylococcus aureus* secreted protein SSL7 (Laursen et al. (2010) Proc. Natl. Acad. Sci. 107:3681-3686).

Methods for determining whether an agent is a C5 antagonist are well known in the art. The C5 antagonists described herein have activity in blocking the generation or activity of the C5a and/or C5b active fragments of a complement component C5 protein (e.g., a human C5 protein). Through this blocking effect, the C5 antagonists inhibit, e.g., the proinflammatory effects of C5a and the generation of the C5b-9 membrane attack complex (MAC) at the surface of a cell.

Inhibition of human complement component C5 can also reduce the cell-lysing ability of complement in a subject's body fluids. Such reductions of the cell-lysing ability of complement present in the body fluid(s) can be measured by methods well known in the art such as, for example, by a conventional hemolytic assay such as the hemolysis assay described by Kabat and Mayer (eds.), "Experimental Immunochemistry, 2nd Edition," 135-240, Springfield, IL, CC Thomas (1961), pages 135-139, or a conventional variation of that assay such as the chicken erythrocyte hemolysis method as described in, e.g., Hillmen et al. (2004) N Engl J Med 350(6):552. Methods for determining whether an agent inhibits the cleavage of human C5 into forms C5a and C5b are known in the art and described in, e.g., Moongkarndi et al. (1982) Immunobiol. 162:397; Moongkarndi et al. (1983) Immunobiol. 165:323; Isenman et al. (1980) J Immunol. 124(1):326-31; Thomas et al. (1996) Mol. Immunol. 33(17-18):1389-401; and Evans et al. (1995) Mol. Immunol. 32(16):1183-95. For example, the concentration and/or physiologic activity of C5a and C5b in a body fluid can be measured by methods well known in the art. Methods for measuring C5a concentration or activity include, e.g., chemotaxis assays, RIAs, or ELISAs (see, e.g., Ward and Zvaifler (1971) J Clin Invest. 50(3):606-16 and Wurzner et al. (1991) Complement Inflamm. 8:328-340). For C5b, hemolytic assays or assays for soluble C5b-9 as discussed herein can be used. Other assays known in the art can also be used. Using assays of these or other suitable types, agents capable of inhibiting human complement component C5 can be screened.

Immunological techniques such as, but not limited to, ELISA can be used to measure the protein concentration of C5 and/or its split products to determine the ability of a test compound to inhibit conversion of C5 into biologically active products. In some embodiments, C5a generation is measured. In some embodiments, C5b-9 neoepitope-specific antibodies are used to detect the formation of terminal complement.

Hemolytic assays can be used to determine the inhibitory activity of a putative C5 antagonist on complement activation. In order to determine the effect of a C5 antagonist on classical complement pathway-mediated hemolysis in a serum test solution *in vitro,* for example, sheep erythrocytes coated with hemolysin or chicken erythrocytes sensitized with anti-chicken erythrocyte antibody are used as target cells. The percentage of lysis is normalized by considering 100% lysis equal to the lysis occurring in the absence of the inhibitor. In some embodiments, the classical complement pathway is activated by a human IgM antibody, for example, as utilized in the Wieslab® Classical Pathway Complement Kit (Wieslab® COMPL CP310, Euro-Diagnostica, Sweden). Briefly, the test serum is incubated with a test compound in the presence of a human IgM antibody. The amount of C5b-9 that is generated is measured by contacting the mixture with an enzyme conjugated anti-C5b-9 antibody and a fluorogenic substrate and measuring the absorbance at the appropriate wavelength. As a control, the test serum is incubated in the absence of the putative C5 antagonist. In some embodiments, the test serum is a C5-deficient serum reconstituted with a C5 polypeptide.

To determine the effect of putative C5 antagonist on alternative pathway-mediated hemolysis, unsensitized rabbit or guinea pig erythrocytes are used as the target cells. In some embodiments, the serum test solution is a C5-deficient serum reconstituted with a C5 polypeptide. The percentage of lysis is normalized by considering 100% lysis equal to the lysis occurring in the absence of the inhibitor. In some embodiments, the alternative complement pathway is activated by lipopolysaccharide molecules, for example, as utilized in the Wieslab® Alternative Pathway Complement Kit (Wieslab® COMPL AP330, Euro-Diagnostica, Sweden). Briefly, the test serum is incubated with a test compound in the presence of lipopolysaccharide. The amount of C5b-9 that is generated is measured by contacting the mixture with an enzyme conjugated anti-C5b-9 antibody and a fluorogenic substrate and measuring the fluorescence at the appropriate wavelength. As a control, the test serum is incubated in the absence of the test compound.

In some embodiments, C5 activity, or inhibition thereof, is quantified using a CH50eq assay. The CH50eq assay is a method for measuring the total classical complement activity in serum. This test is a lytic assay, which uses antibody-sensitized erythrocytes as the activator of the classical complement pathway and various dilutions of the test serum to determine the amount required to give 50% lysis (CH50). The percent hemolysis can be determined, for example, using a spectrophotometer. The CH50eq assay provides an indirect measure of terminal complement complex (TCC) formation, since the TCC themselves are directly responsible for the hemolysis that is measured.

The assay is well known and commonly practiced by those of skill in the art. Briefly, to activate the classical complement pathway, undiluted serum samples (e.g., reconstituted human serum samples) are added to microassay wells containing the antibody-sensitized erythrocytes to thereby generate TCC. Next, the activated sera are diluted in microassay wells, which are coated with a capture reagent (e.g., an antibody that binds to one or more components of the TCC). The TCC present in the activated samples bind to the monoclonal antibodies coating the surface of the microassay wells. The wells are washed and to each well is added a detection reagent that is detectably labeled and recognizes the bound TCC. The detectable label can be, e.g., a fluorescent label or an enzymatic label. The assay results are expressed in CH50 unit equivalents per milliliter (CH50 U Eq/mL).

Methods for determining the half-life of a therapeutic agent (e.g., a C5 antagonist such as an anti-C5 antibody), as well as changes in half-life (e.g., increases in half-life), are well known in the art. See, e.g., International patent application publication no. WO 2010/151526; International patent application publication no. WO 98/23289; International patent application publication no. WO 97/34631; U.S. patent no. 6,277,375; International patent application publication nos. WO 93/15199, WO 93/15200, and WO 01/77137; European Patent No. EP 413 622; and U.S. patent application publication no. 20110111406. See also Hinton et al. (2006) J Immunol 176:346-356.

Methods for Treatment

The methods described herein include administering to a subject (e.g., a human) a compound that reduces the concentration of an antigen to which a therapeutic agent binds (e.g., human complement component C5) and, in the context of the reduced concentration of the antigen (e.g., C5), administering to the human the therapeutic agent (e.g., a C5 antagonist such as an anti-C5 antibody).

The compounds and agents (e.g., C5 antagonists) described herein can be administered to a subject, e.g., a human subject, using a variety of methods that depend, in part, on the route of administration. The route can be, e.g., intravenous injection or infusion (IV), subcutaneous injection (SC), intraperitoneal (IP) injection, or intramuscular injection (IM).

Administration can be achieved by, e.g., local infusion, injection, or by means of an implant. The implant can be of a porous, non-porous, or gelatinous material, including membranes, such as sialastic membranes, or fibers. The implant can be configured for sustained or periodic release of the composition to the subject. See, e.g., U.S. Patent Application Publication No. 20080241223; U.S. Patent Nos. 5,501,856; 4,863,457; and 3,710,795; EP488401; and EP430539, the disclosures of each of which are incorporated herein by reference in their entirety. A composition described herein (e.g., a compound and/or a C5 antagonist) can be delivered to the subject by way of an implantable device based on, e.g., diffusive, erodible, or convective systems, e.g., osmotic pumps, biodegradable implants, electrodiffusion systems, electroosmosis systems, vapor pressure pumps, electrolytic pumps, effervescent pumps, piezoelectric pumps, erosion-based systems, or electromechanical systems.

In some embodiments, a composition described herein is therapeutically delivered to a subject by way of local administration. As used herein, "local administration" or "local delivery," refers to delivery that does not rely upon transport of the composition or agent to its intended target tissue or site via the vascular system. For example, the composition may be delivered by injection or implantation of the composition or agent or by injection or implantation of a device containing the composition or agent. Following local administration in the vicinity of a target tissue or site, a C5 antagonist, e.g., may diffuse to the intended target tissue or site.

In some embodiments, a composition described herein can be locally administered to a joint (e.g., an articulated joint). For example, in embodiments where the disorder is arthritis, a therapeutically appropriate composition can be administered directly to a joint (e.g., into a joint space) or in the vicinity of a joint. Examples of intraarticular joints to which a composition described herein can be locally administered include, e.g., the hip, knee, elbow, wrist, sternoclavicular, temperomandibular, carpal, tarsal, ankle, and any other joint subject to arthritic conditions. A composition described herein can also be administered to bursa such as, e.g., acromial, bicipitoradial, cubitoradial, deltoid, infrapatellar, ischial, and any other bursa known in the art of medicine.

In some embodiments, a composition described herein can be locally administered to the eye. As used herein, the term "eye" refers to any and all anatomical tissues and structures associated with an eye. The eye has a wall composed of three distinct layers: the outer sclera, the middle choroid layer, and the inner retina. The chamber behind the lens is filled with a gelatinous fluid referred to as the vitreous humor. At the back of the eye is the retina, which detects light. The cornea is an optically transparent tissue, which conveys images to the back of the eye. The cornea includes one pathway for the permeation of drugs into the eye. Other anatomical tissue structures associated with the eye include the lacrimal drainage system, which includes a secretory system, a distributive system and an excretory system. The secretory system comprises secretors that are stimulated by blinking and temperature change due to tear evaporation and reflex secretors that have an efferent parasympathetic nerve supply and secrete tears in response to physical or emotional stimulation. The distributive system includes the eyelids and the tear meniscus around the lid edges of an open eye, which spread tears over the ocular surface by blinking, thus reducing dry areas from developing.

In some embodiments, a composition (e.g., one or both of the compound and a C5 antagonist) described herein is administered to the posterior chamber of the eye. In some embodiments, a composition described herein is administered intravitreally. In some embodiments, a composition described herein is administered trans-sclerally.

In some embodiments, e.g., in embodiments for treatment or prevention of a disorder such as COPD or asthma, a composition (e.g., a C5 antagonist) described herein can be administered to a subject by way of the lung. Pulmonary drug delivery may be achieved by inhalation, and administration by inhalation herein may be oral and/or nasal. Examples of pharmaceutical devices for pulmonary delivery include metered dose inhalers, dry powder inhalers (DPIs), and nebulizers. For example, a composition described herein can be administered to the lungs of a subject by way of a dry powder inhaler. These inhalers are propellant-free devices that deliver dispersible and stable dry powder formulations to the lungs. Dry powder inhalers are well known in the art of medicine and include, without limitation: the TurboHaler® (AstraZeneca; London, England); the AIR® inhaler (Alkermes®; Cambridge, Massachusetts); Rotahaler® (GlaxoSmithKline; London, England); and Eclipse™ (Sanofi-Aventis; Paris, France). See also, e.g., PCT Publication Nos. WO 04/026380, WO 04/024156, and WO 01/78693. DPI devices have been used for pulmonary administration of polypeptides such as insulin and growth hormone. In some embodiments, a composition described herein can be intrapulmonarily administered by way of a metered dose inhaler. These inhalers rely on a propellant to deliver a discrete dose of a compound to the lungs.

In some embodiments, a composition (e.g., a C5 antagonist) described herein can be administered to the lungs of a subject by way of a nebulizer. Nebulizers use compressed air to deliver a compound as a liquefied aerosol or mist. A nebulizer can be, e.g., a jet nebulizer (e.g., air or liquid-jet nebulizers) or an ultrasonic nebulizer. Additional devices and intrapulmonary administration methods are set forth in, e.g., U.S. Patent Application Publication Nos. 20050271660 and 20090110679, the disclosures of each of which are incorporated herein by reference in their entirety.

In some embodiments, the compositions provided herein are present in unit dosage form, which can be particularly suitable for self-administration. For example, in some embodiments the compound that reduces the serum concentration of an antigen (e.g., human C5) can be formulated for self-administration (e.g., self-subcutaneous administration). A formulated product of the present disclosure can be included within a container, typically, for example, a vial, cartridge, prefilled syringe or disposable pen. A doser such as the doser device described in U.S. Patent No. 6,302,855 may also be used, for example, with an injection system of the present disclosure.

An injection system of the present disclosure may employ a delivery pen as described in U.S. Patent No. 5,308,341. Pen devices, most commonly used for self-delivery of insulin to patients with diabetes, are well known in the art. Such devices can comprise at least one injection needle (e.g., a 31 gauge needle of about 5 to 8 mm in length), are typically pre-filled with one or more therapeutic unit doses of a therapeutic solution, and are useful for rapidly delivering the solution to a subject with as little pain as possible.

One medication delivery pen includes a vial holder into which a vial of insulin or other medication may be received. The vial holder is an elongate generally tubular structure with proximal and distal ends. The distal end of the vial holder includes mounting means for engaging a double-ended needle cannula. The proximal end also includes mounting means for engaging a pen body which includes a driver and dose setting apparatus. A disposable medication (e.g., a high concentration solution of a composition described herein) containing vial for use with the prior art vial holder includes a distal end having a pierceable elastomeric septum that can be pierced by one end of a double-ended needle cannula. The proximal end of this vial includes a stopper slidably disposed in fluid tight engagement with the cylindrical wall of the vial. This medication delivery pen is used by inserting the vial of medication into the vial holder. A pen body then is connected to the proximal end of the vial holder. The pen body includes a dose setting apparatus for designating a dose of medication to be delivered by the pen and a driving apparatus for urging the stopper of the vial distally for a distance corresponding to the selected dose. The user of the pen mounts a double-ended needle cannula to the distal end of the vial holder such that the proximal point of the needle cannula pierces the septum on the vial. The patient then selects a dose and operates the pen to urge the stopper distally to deliver the selected dose. The dose selecting apparatus returns to zero upon injection of the selected dose. The patient then removes and discards the needle cannula, and keeps the medication delivery pen in a convenient location for the next required medication administration. The medication in the vial will become exhausted after several such administrations of medication. The patient then separates the vial holder from the pen body. The empty vial may then be removed and discarded. A new vial can be inserted into the vial holder, and the vial holder and pen body can be reassembled and used as explained above. Accordingly, a medication delivery pen generally has a drive mechanism for accurate dosing and ease of use.

A dosage mechanism such as a rotatable knob allows the user to accurately adjust the amount of medication that will be injected by the pen from a prepackaged vial of medication. To inject the dose of medication, the user inserts the needle under the skin and depresses the knob once as far as it will depress. The pen may be an entirely mechanical device or it may be combined with electronic circuitry to accurately set and/or indicate the dosage of medication that is injected into the user. See, e.g., U.S. Patent No. 6,192,891.

In some embodiments, the needle of the pen device is disposable and the kits include one or more disposable replacement needles. Pen devices suitable for delivery of any one of the presently featured compositions are also described in, e.g., U.S. patent nos. 6,277,099; 6,200,296; and 6,146,361, the disclosures of each of which are incorporated herein by reference in their entirety. A microneedle-based pen device is described in, e.g., U.S. patent no. 7,556,615, the disclosure of which is incorporated herein by reference in its entirety. See also the Precision Pen Injector (PPI) device, Molly™, manufactured by Scandinavian Health Ltd.

The present disclosure also presents controlled-release or extended-release formulations suitable for chronic and/or self-administration of a medication such as a composition (e.g., a compound or a C5 antagonist) described herein. The various formulations can be administered to a patient in need of treatment with the medication as a bolus or by continuous infusion over a period of time.

In some embodiments, a high concentration composition (e.g., a high concentration compound or C5 antagonist) described herein is formulated for sustained-release, extended-release, timed-release, controlled-release, or continuous-release administration. In some embodiments, depot formulations are used to administer the composition to the subject in need thereof. In this method, the composition is formulated with one or more carriers providing a gradual release of active agent over a period of a number of hours or days. Such formulations are often based upon a degrading matrix which gradually disperses in the body to release the active agent.

As noted above, in some embodiments the compound and the therapeutic agent (e.g., the C5 antagonist) can be administered to a human by different routes. For example, the compound such as an siRNA that targets C5 can be administered subcutaneously (e.g., once weekly) and the C5 antagonist (e.g., an anti-C5 antibody such as eculizumab) can be administered intravenously (e.g., once monthly or once every two months).

A suitable dose of a given composition described herein, which dose is capable of treating or preventing a disorder in a subject, can depend on a variety of factors including, e.g., the age, sex, and weight of a subject to be treated and the particular inhibitor compound used. Other factors can include, e.g., other medical disorders concurrently or previously affecting the subject, the general health of the subject, the genetic disposition of the subject, diet, time of administration, rate of excretion, drug combination, and any other additional therapeutics that are administered to the subject. It should also be understood that a specific dosage and treatment regimen for any particular subject will also depend upon the judgment of the treating medical practitioner (e.g., doctor or nurse).

A composition described herein can be administered as a fixed dose, or in a milligram per kilogram (mg/kg) dose. In some embodiments, the dose can also be chosen to reduce or avoid production of antibodies or other host immune responses against a compound or a therapeutic agent (such as a C5 antagonist). While in no way intended to be limiting, exemplary dosages of an antibody, such as a composition described herein include, e.g., 1-1000 mg/kg, 1-100 mg/kg, 0.5-50 mg/kg, 0.1-100 mg/kg, 0.5-25 mg/kg, 1-20 mg/kg, and 1-10 mg/kg. Exemplary dosages of a composition described herein include, without limitation, 0.1 mg/kg, 0.5 mg/kg, 1.0 mg/kg, 2.0 mg/kg, 4 mg/kg, 8 mg/kg, or 20 mg/kg.

A pharmaceutical composition can include a therapeutically effective amount of a composition described herein. Such effective amounts can be readily determined by one of ordinary skill in the art based, in part, on the effect of the administered composition, or the combinatorial effect of the composition and one or more additional active agents, if more than one agent is used. A therapeutically effective amount of a composition described herein can also vary according to factors such as the disease state, age, sex, and weight of the individual, and the ability of the composition (and one or more additional active agents) to elicit a desired response in the individual, e.g., amelioration of at least one condition parameter, e.g., amelioration of at least one symptom of the complement-mediated disorder. For example, a therapeutically effective amount of a composition described herein can inhibit (lessen the severity of or eliminate the occurrence of) and/or prevent a particular disorder, and/or any one of the symptoms of the particular disorder known in the art or described herein. A therapeutically effective amount is also one in which any toxic or detrimental effects of the composition are outweighed by the therapeutically beneficial effects.

Suitable human doses of any of the compositions described herein (e.g., a compound or a C5 antagonist) can further be evaluated in, e.g., Phase I dose escalation studies. See, e.g., van Gurp et al. (2008) Am J Transplantation 8(8):1711-1718; Hanouska et al. (2007) Clin Cancer Res 13(2, part 1):523-531; and Hetherington et al. (2006) Antimicrobial Agents and Chemotherapy 50(10): 3499-3500. For example, suitable dosages and/or frequency of dosages of a compound required to reduce serum C5 concentration can be determined using such methods.

Toxicity and therapeutic efficacy of such compositions (e.g., the compound or the C5 antagonist) can be determined by known pharmaceutical procedures in cell cultures or experimental animals (e.g., animal models of any of the complement-mediated disorders described herein). These procedures can be used, e.g., for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Compositions described herein that exhibit a high therapeutic index are preferred. While compositions that exhibit toxic side effects may be used, care should be taken to design a delivery system that targets such compounds to the site of affected tissue and to minimize potential damage to normal cells and, thereby, reduce side effects.

Data obtained from cell culture assays and animal studies can be used in formulating a range of dosage for use in humans. The dosage of the composition described herein lies generally within a range of circulating concentrations of the compositions that include the $ED_{50}$ with little or no toxicity. The dosage may vary within this range depending upon the dosage form employed and the route of administration utilized. For a composition described herein, the therapeutically effective dose can be estimated initially from cell culture assays. A dose can be formulated in animal models to achieve a circulating plasma concentration range that includes the $IC_{50}$ (i.e., the concentration of the antibody which achieves a half-maximal inhibition of symptoms) as determined in cell culture. Such information can be used to more accurately determine useful doses in humans. Levels in plasma may be measured, for example, by high performance liquid chromatography. In some embodiments, e.g., where local administration (e.g., to the eye or a joint) is desired, cell culture or animal modeling can be used to determine a dose required to achieve a therapeutically effective concentration within the local site.

In some embodiments, the methods can be performed in conjunction with other therapies for complement-associated disorders. For example, the composition can be administered to a subject at the same time, prior to, or after, plasmapheresis, IVIG therapy, or plasma exchange. See, e.g., Appel et al. (2005) J Am Soc Nephrol 16:1392-1404. In some embodiments, the composition can be administered to a subject at the same time, prior to, or after, a kidney transplant.

A "subject," as used herein, can be any mammal. For example, a subject can be a human, a non-human primate (e.g., orangutan, gorilla, macaque, baboon, or chimpanzee), a horse, a cow, a pig, a sheep, a goat, a dog, a cat, a rabbit, a guinea pig, a gerbil, a hamster, a rat, or a mouse. In some embodiments, the subject is an infant (e.g., a human infant).

As used herein, a subject "in need of prevention," "in need of treatment," or "in need thereof," refers to one, who by the judgment of an appropriate medical practitioner (e.g., a doctor, a nurse, or a nurse practitioner in the case of humans; a veterinarian in the case of non-human mammals), would reasonably benefit from a given treatment.

The term "preventing" is art-recognized, and when used in relation to a condition, is well understood in the art, and includes administration of a composition which reduces the frequency of, or delays the onset of, symptoms of a medical condition in a subject relative to a subject which does not receive a composition described herein. Thus, prevention of a complement-associated disorder such as asthma includes, for example, reducing the extent or frequency of coughing, wheezing, or chest pain in a population of patients receiving a prophylactic treatment relative to an untreated control population, and/or delaying the occurrence of coughing or wheezing in a treated population versus an untreated control population, e.g., by a statistically and/or clinically significant amount.

As described above, the compositions described herein (e.g., C5 antagonists such an anti-C5 antibodies) can be used to treat a variety of complement-associated disorders such as, but not limited to: rheumatoid arthritis (RA); lupus nephritis; ischemia-reperfusion injury; atypical hemolytic uremic syndrome (aHUS); typical or infectious hemolytic uremic syndrome (tHUS); dense deposit disease (DDD); paroxysmal nocturnal hemoglobinuria (PNH); multiple sclerosis (MS); macular degeneration (e.g., age-related macular degeneration (AMD)); hemolysis, elevated liver enzymes, and low platelets (HELLP) syndrome; sepsis; dermatomyositis; diabetic retinopathy; thrombotic thrombocytopenic purpura (TTP); spontaneous fetal loss; Pauci-immune vasculitis; epidermolysis bullosa; recurrent fetal loss; and traumatic brain injury. See, e.g., Holers (2008) Immunological Reviews 223:300-316 and Holers and Thurman (2004) Molecular Immunology 41:147-152. In some embodiments, the complement-mediated disorder is a complement-mediated vascular disorder such as, but not limited to, a cardiovascular disorder, myocarditis, a cerebrovascular disorder, a peripheral (e.g., musculoskeletal) vascular disorder, a renovascular disorder, a mesenteric/enteric vascular disorder, revascularization to transplants and/or replants, vasculitis, Henoch-Schonlein purpura nephritis, systemic lupus erythematosus-associated vasculitis, vasculitis associated with rheumatoid arthritis, immune complex vasculitis, organ or tissue transplantation, Takayasu's disease, capillary leak syndrome, dilated cardiomyopathy, diabetic angiopathy, thoracic-abdominal aortic aneurysm, Kawasaki's disease (arteritis), venous gas embolus (VGE), and restenosis following stent placement, rotational atherectomy, and percutaneous transluminal coronary angioplasty (PTCA). (See, e.g., U.S. patent application publication no. 20070172483.) In some embodiments, the complement-associated disorder is myasthenia gravis, cold-agglutinin disease (CAD), paroxysmal cold hemoglobinuria (PCH), dermatomyositis, scleroderma, warm autoimmune hemolytic anemia, Graves' disease, Hashimoto's thyroiditis, type I diabetes, psoriasis, pemphigus, autoimmune hemolytic anemia (AIHA), idiopathic thrombocytopenic purpura (ITP), Goodpasture's syndrome, antiphospholipid syndrome (APS), Degos disease, and catastrophic APS (CAPS).

In some embodiments, a composition described herein can be used to treat an inflammatory disorder such as, but not limited to, RA (above), inflammatory bowel disease, sepsis (above), septic shock, acute lung injury, disseminated intravascular coagulation (DIC), or Crohn's disease. In some embodiments, the second anti-inflammatory agent can be one selected from the group consisting of NSAIDs, corticosteroids, methotrexate, hydroxychloroquine, anti-TNF agents such as etanercept and infliximab, a B cell depleting agent such as rituximab, an interleukin-1 antagonist, or a T cell costimulatory blocking agent such as abatacept.

In some embodiments, the complement-associated disorder is a complement-associated neurological disorder such as, but not limited to, amyotrophic lateral sclerosis (ALS), brain injury, Alzheimer's disease, and chronic inflammatory demyelinating neuropathy.

Complement-associated disorders also include complement-associated pulmonary disorders such as, but not limited to, asthma, bronchitis, a chronic obstructive pulmonary disease (COPD), an interstitial lung disease, α-1 anti-trypsin deficiency, emphysema, bronchiectasis, bronchiolitis obliterans, alveolitis, sarcoidosis, pulmonary fibrosis, and collagen vascular disorders.

In some embodiments, a composition described herein is administered to a subject to treat, prevent, or ameliorate at least one symptom of a complement-associated inflammatory response (e.g., the complement-associated inflammatory response aspect of a complement-associated disorder) in a subject. For example, a composition can be used to treat, prevent, and/or ameliorate one or more symptoms associated with a complement-associated inflammatory response such as graft rejection/graft-versus-host disease (GVHD), reperfusion injuries (e.g., following cardiopulmonary bypass or a tissue transplant), and tissue damage following other forms of traumatic injury such as a burn (e.g., a severe burn), blunt trauma, spinal injury, or frostbite. See, e.g., Park et al. (1999) Anesth Analg 99(1):42-48; Tofukuji et al. (1998) J Thorac Cardiovasc Surg 116(6):1060-1068; Schmid et al. (1997) Shock 8(2):119-124; and Bless et al. (1999) Am J Physiol 276(1):L57-L63.

Monitoring a subject (e.g., a human patient) for an improvement in a disorder (e.g., sepsis, severe burn, RA, lupus nephritis, Goodpasture syndrome, or asthma), as defined herein, means evaluating the subject for a change in a disease parameter, e.g., an improvement in one or more symptoms of a given disorder. The symptoms of many of the above disorders (e.g., complement-associated disorders) are well known in the art of medicine. In some embodiments, the evaluation is performed at least one (1) hour, e.g., at least 2, 4, 6, 8, 12, 24, or 48 hours, or at least 1 day, 2 days, 4 days, 10 days, 13 days, 20 days or more, or at least 1 week, 2 weeks, 4 weeks, 10 weeks, 13 weeks, 20 weeks or more, after an administration of a composition described herein. The subject can be evaluated in one or more of the following periods: prior to beginning of treatment; during the treatment; or after one or more elements of the treatment have been administered. Evaluation can include evaluating the need for further treatment, e.g., evaluating whether a dosage, frequency of administration, or duration of treatment should be altered. It can also include evaluating the need to add or drop a selected therapeutic modality, e.g., adding or dropping any of the treatments for a complement-associated disorder described herein.

The compound that reduces serum C5 concentration can be administered to a human in an amount and with a frequency sufficient to reduce serum C5 concentration. In the context of reduced serum C5 concentration, a therapeutically effective amount of a C5 antagonist is administered to the human, e.g., in an amount and with a frequency sufficient to maintain serum complement activity below 20% of the level of complement activity in a human in the absence of the C5 antagonist or any other complement antagonist. In some embodiments, the compound and C5 antagonist can be administered at the same time.

The following examples are intended to illustrate, not limit, the invention.

## Examples

### Example 1. Treatment of a human afflicted with PNH

A human patient is identified by a medical practitioner as having PNH. The patient is one treated with an anti-C5 antibody under the following dose schedule: (i) 600 mg each week for four weeks; (ii) 900 mg 1 week later; and (iii) 900 mg on a biweekly basis thereafter. The patient is then placed on a new therapeutic regimen. A human C5-specific siRNA is chronically administered to the patient to reduce the concentration of C5 in the serum of the patient. In the context of reduced serum C5 levels, the anti-C5 antibody is administered to the patient at a dose of 600 mg every three months. The patient and medical practitioner continue to observe that the patient continues to do as well under the new therapeutic regimen as under the previous regimen or may even observe a substantial improvement in PNH symptoms and pathology under the new regimen.

### Example 2. Impact of C5 on the clearance of an anti-C5 antibody

To understand the potential impact of antigen-mediated clearance on the overall clearance rate of an anti-human C5 (hC5) antibody, the following experiments were performed using the human neonatal Fc receptor (hFcRn) transgenic mouse model (the mice lack endogenous FcRn but are transgenic for hFcRn (B6.Cg-Fcgrt$^{tm1Dcr}$ Tg(FCGRT)32Dcr/DcrJ; Stock Number 014565, Jackson Laboratories, Bar Harbor, Maine)). The transgenic hFcRn model has been described in, e.g., Petkova et al. (2006) Int Immunology 18(12):1759-1769; Oiao et al. (2008) Proc Natl Acad Sci USA 105(27):9337-9342; and Roopenian et al. (2010) Methods Mol Biol 602:93-104.

A single dose of 50 μg of the anti-hC5 antibody in 200 μL of phosphate buffered saline (PBS) was administered by intravenous (i.v.) injection to each of six hFcRn transgenic mice. Alternatively, the anti-hC5 antibody was pre-incubated in a 4:1 or 2:1 molar ratio of human C5 (Complement Technology Inc., Catalog Number: A120) to antibody at 4°C overnight and the mixtures were administered to the animals. Yet another group of mice were treated with the 4:1 hC5/anti-hC5 antibody mixture on day 0 (the first day of the experiment) followed by an additional 250 μg of hC5 administered by intravenous administration on day 1.

Blood samples of approximately 100 μL were collected from each of the mice at days one, three, seven, 14, 21, 28, and 35 following the administration. The concentration of the anti-hC5 antibody in serum was measured by ELISA.

Antibody serum half-life was calculated using the following formula:

$$Halflife = T \times \frac{\ln 2}{\ln \frac{A_0}{A_t}}$$

where: T = Time elapsed , $A_o$ = Original serum concentration of the antibody (concentration at day 1 in the present study) and $A_t$ = Amount of the antibody remaining after elapsed time T (minimal detectable concentration or the last bleeding time point (day 35) in the present study).

The results of the experiment are as follows. In the absence of hC5, the half-life of the anti-hC5 antibody in the hFcRn mouse model was 13.49 ± 0.93 days. The half-life of the anti-hC5 antibody mixed with a 2-fold molar excess of hC5 was 9.58 ± 1.24 days. Mixing the anti-hC5 antibody with a 4-fold molar excess of hC5 resulted in an antibody half-life of 5.77 ± 1.86 days. The additional subsequent dose of hC5 to mice administered the 4:1 hC5/anti-hC5 antibody mixture reduced the half-life of the antibody to 4.55 ± 1.02 days.

These results indicate that the presence of higher amounts of hC5 markedly shorten the half-life of an anti-hC5 antibody. The half-life of the antibody depends significantly on the amount of free antibody in circulation. The results suggest that a reduced concentration of human C5 would significantly reduce the impact of antigen-mediated antibody clearance and thus increase the half-life of an anti-hC5 antibody in a human.

While the present disclosure has been described with reference to the specific embodiments thereof, it should be understood by those skilled in the art that various changes may be made and equivalents may be substituted without departing from the true spirit and scope of the disclosure. In addition, many modifications may be made to adapt a particular situation, material, composition of matter, process, process step or steps, to the objective, spirit and scope of the present disclosure. All such modifications are intended to be within the scope of the disclosure.

ASPECTS OF THE INVENTION

1. A method for increasing the half-life of a C5 antagonist in the serum of a human, the method comprising:

    (i) administering to the human a compound that reduces the concentration of complement component C5 in the serum of the human to thereby reduce the C5 concentration in the serum of the human; and
    (ii) administering to the human the C5 antagonist, wherein a reduced C5 concentration in the serum of the human increases the serum half-life of the C5 antagonist administered to the human.

2. The method of aspect 1, wherein the human has, is suspected of having, or is at risk for developing, a complement-associated disorder.

3. A method for decreasing the frequency at which a therapeutically effective amount of a C5 antagonist must be administered to a human having, suspected of having, or at risk for developing, a complement-associated disorder, the method comprising:

    (i) administering to the human a compound that reduces the concentration of complement component C5 in the serum of the human to thereby reduce the C5 concentration in the serum of the human; and
    (ii) administering to the human a therapeutically effective amount of the C5 antagonist, wherein a reduced C5 concentration in the serum of the human decreases the frequency at which a therapeutically effective amount of a C5 antagonist must be administered to the human.

4. A method for decreasing the dosage of a C5 antagonist required for therapeutic efficacy in a human having, suspected of having, or at risk for developing, a complement-associated disorder, the method comprising:

    (i) administering to the human a compound that reduces the concentration of complement component C5 in the serum of the human to thereby reduce the C5 concentration in the serum of the human; and
(ii) administering to the human a therapeutically effective amount of the C5 antagonist, wherein a reduced C5 concentration in the serum of the human decreases the dosage of a C5 antagonist required for therapeutic efficacy in the human.

5. The method according to any one of aspects 1-4, wherein the compound reduces the level of expression of complement component C5 by one or more cells in the human.

6. The method according to aspect 5, wherein the compound inhibits transcription of a human complement component C5 gene.

7. The method according to aspect 5, wherein the compound inhibits translation of an mRNA encoding human complement component C5.

8. The method according to aspect 5, wherein the compound reduces the stability of an mRNA encoding human complement component C5.

9. The method according to aspect 5, wherein the compound is an siRNA specific for mRNA encoding human complement component C5.

10. The method according to aspect 5, wherein the compound is an antisense nucleic acid complementary to a mRNA encoding human complement component C5.

11. The method according to any one of aspects 1 to 10, wherein the C5 antagonist is selected from the group consisting of: MB 12/22, MB12/22-RGD, ARC187, ARC 1905, SSL7, and OmCI.

12. The method according to any one of aspects 1 to 10, wherein the C5 antagonist is an antibody, or an antigen-binding fragment thereof, that binds to complement component C5 and inhibits the cleavage of C5 into fragments C5a and C5b.

13. The method according to aspect 12, wherein the antibody or antigen-binding fragment thereof is selected from the group consisting of a polyclonal antibody, a recombinant antibody, a diabody, a chimerized or chimeric antibody, a deimmunized antibody, a fully human antibody, a single chain antibody, a domain antibody, an Fv fragment, an Fd fragment, an Fab fragment, an Fab' fragment, and an $F(ab')_2$ fragment.

14. The method according to aspect 12 or 13, wherein the antibody or antigen-binding fragment thereof is a bispecific antibody.

15. The method according to aspect 14, wherein the antibody or antigen-binding fragment thereof is a DVD-Ig antibody.

16. The method according to aspect 12, wherein the antibody is eculizumab.

17. The method according to aspect 12, wherein the antigen-binding fragment is pexelizumab.

18. The method according to any one of aspects 1 to 17, wherein the compound reduces the serum concentration of C5 by at least 10%.

19. The method according to any one of aspects 1 to 18, wherein the compound reduces the serum concentration of C5 by at least 20%.

20. The method according to any one of aspects 1 to 19, wherein the compound reduces the serum concentration of C5 by at least 40%.

21. The method according to any one of aspects 1 to 20, wherein the compound is chronically administered to the human.

22. The method according to aspect 21, wherein the compound is administered to the human at least once weekly for at least three weeks.

23. The method according to aspect 21, wherein the compound is administered to the human at least once weekly for at least six weeks.

24. The method according to aspect 21, wherein the compound is administered to the human at least once weekly for at least six months.

25. The method according to any one of aspects 1 to 24, wherein the compound is subcutaneously administered to the human.

26. The method according to any one of aspects 1 to 25, wherein the C5 antagonist is chronically administered to the human.

27. The method according to any one of aspects 1 to 26, wherein the C5 antagonist and the compound are both chronically administered to the human.

28. The method according to any one of aspects 1 to 27, wherein the C5 antagonist and the compound are administered to the human using different routes of administration.

29. The method according to any one of aspects 1 to 28, wherein the C5 antagonist and the compound are administered to the human under different dosing schedules.

30. The method according to any one of aspects 1 to 29, wherein the serum half-life of the C5 antagonist is increased 2-fold as compared to the half-life in the absence of administering the compound.

31. The method according to any one of aspects 1 to 29, wherein the serum half-life of the C5 antagonist is increased 5-fold as compared to the half-life in the absence of administering the compound.

32. The method according to any one of aspects 1 to 29, wherein the serum half-life of the C5 antagonist is increased 10-fold as compared to the half-life in the absence of administering the compound.

33. The method according to any one of aspects 1 to 32, wherein the therapeutically effective amount of the C5 antagonist administered to the human having a reduced C5 concentration is less than the therapeutically effective amount of the C5 antagonist required without the reduction in C5 concentration.

34. The method according to any one of aspects 1 to 33, wherein the frequency of administration of the C5 antagonist to the human having a reduced C5 concentration is less often than the frequency of administration of the C5 antagonist to the human required without the reduction in C5 concentration.

35. The method according to aspect 34, wherein the C5 antagonist is an anti-C5 antibody and the frequency of administration of the C5 antagonist to the human having a reduced C5 concentration is no more frequently than once monthly.

36. The method according to aspect 34, wherein the C5 antagonist is an anti-C5 antibody and the frequency of administration of the C5 antagonist to the human having a reduced C5 concentration is no more frequently than once every two months.

37. The method according to any one of aspects 2 to 36, wherein the complement-associated disorder is selected from the group consisting of paroxysmal nocturnal hemoglobinuria (PNH), atypical hemolytic-uremic syndrome (aHUS), shiga toxin *E. coli*-related hemolytic uremic syndrome (STEC-HUS), dense deposit disease (DDD), C3 nephropathy, myasthenia gravis, neuromyelitis optica, cold agglutinin disease (CAD), antineutrophil cytoplasm antibody (ANCA)-associated vasculitis (AAV), asthma, age-related macular degeneration (AMD), transplant rejection, Goodpasture's syndrome, glomerulonephritis, vasculitis, rheumatoid arthritis, dermatitis, systemic lupus erythematosus (SLE), Guillain-Barré syndrome (GBS), dermatomyositis, psoriasis, Graves' disease, Hashimoto's thyroiditis, type I diabetes, pemphigus, autoimmune hemolytic anemia (AIHA), idiopathic thrombocytopenic purpura (ITP), lupus nephritis, ischemia-reperfusion injury, thrombotic thrombocytopenic purpura (TTP), Pauci-immune vasculitis, epidermolysis bullosa, multiple sclerosis, spontaneous fetal loss, recurrent fetal loss, traumatic brain injury, injury resulting from myocardial infarction, cardiopulmonary bypass and hemodialysis, and hemolysis, elevated liver enzymes, and low platelets (HELLP) syndrome.

38. The method according to any one of aspects 1 to 37 wherein the C5 antagonist is depleted at least in part by antigen-mediated clearance.

39. A method for treating a human afflicted with a complement-associated disorder, the method comprising administering to the human a therapeutically-effective amount of an antagonist of complement component C5, wherein the human has a reduced serum concentration of C5, relative to the normal serum concentration of C5, as the result of the prior administration to the patient of a compound that reduces the serum concentration of C5.

40. The method according to aspect 39, wherein the compound inhibits transcription of a human complement component C5 gene.

41. The method according to aspect 39, wherein the compound inhibits translation of an mRNA encoding human complement component C5.

42. The method according to aspect 39, wherein the compound reduces the stability of an mRNA encoding human complement component C5.

43. The method according to aspect 39, wherein the compound is an siRNA specific for mRNA encoding human complement component C5.

44. The method according to aspect 39, wherein the compound is an antisense nucleic acid complementary to a mRNA encoding human complement component C5.

45. The method according to any one of aspects 39 to 44, wherein the C5 antagonist is selected from the group consisting of: MB12/22, MB12/22-RGD, ARC187, ARC1905, SSL7, and OmCI.

46. The method according to any one of aspects 39 to 44, wherein the C5 antagonist is an antibody, or an antigen-binding fragment thereof, that binds to complement component C5 and inhibits the cleavage of C5 into fragments C5a and C5b.

47. The method according to aspect 46, wherein the antibody or antigen-binding fragment thereof is selected from the group consisting of a polyclonal antibody, a recombinant antibody, a diabody, a chimerized or chimeric antibody, a deimmunized antibody, a fully human antibody, a single chain antibody, a domain antibody, an Fv fragment, an Fd fragment, an Fab fragment, an Fab' fragment, and an $F(ab')_2$ fragment.

48. The method according to aspect 46, wherein the antibody is eculizumab.

49. The method according to aspect 46, wherein the antigen-binding fragment is pexelizumab.

50. The method according to any one of aspects 39 to 49, wherein the compound reduces the serum concentration of C5 by at least 10%.

51. The method according to any one of aspects 39 to 49, wherein the compound reduces the serum concentration of C5 by at least 20%.

52. The method according to any one of aspects 39 to 49, wherein the compound reduces the serum concentration of C5 by at least 40%.

53. The method according to any one of aspects 39 to 52, wherein the compound is chronically administered to the human.

54. The method according to aspect 53, wherein the compound is administered to the human at least once weekly for at least three weeks.

55. The method according to aspect 53, wherein the compound is administered to the human at least once weekly for at least six weeks.

56. The method according to aspect 53, wherein the compound is administered to the human at least once weekly for at least six months.

57. The method according to any one of aspects 39 to 56, wherein the compound is subcutaneously administered to the human.

58. The method according to any one of aspects 39 to 57, wherein the C5 antagonist is chronically administered to the human.

59. The method according to any one of aspects 39 to 58, wherein the C5 antagonist and the compound are both chronically administered to the human.

60. The method according to any one of aspects 39 to 59, wherein the C5 antagonist and the compound are administered to the human using different routes of administration.

61. The method according to any one of aspects 39 to 60, wherein the C5 antagonist and the compound are administered to the human under different dosing schedules.

62. The method according to any one of aspects 39 to 61, wherein the serum half-life of the C5 antagonist is increased at least 2-fold as compared to the half-life in the absence of administering the compound.

63. The method according to any one of aspects 39 to 61, wherein the serum half-life of the C5 antagonist is increased at least 5-fold as compared to the half-life in the absence of administering the compound.

64. The method according to any one of aspects 39 to 61, wherein the serum half-life of the C5 antagonist is increased at least 10-fold as compared to the half-life in the absence of administering the compound.

65. The method according to any one of aspects 39 to 64, wherein the therapeutically effective amount of the C5 antagonist administered to the human having a reduced C5 concentration is less than the therapeutically effective amount of the C5 antagonist required without the reduction in C5 concentration.

66. The method according to any one of aspects 39 to 64, wherein the frequency of administration of the C5 antagonist to the human having a reduced C5 concentration is less often than the frequency of administration of the C5 antagonist to the human required without the reduction in C5 concentration.

67. The method according to aspect 66, wherein the C5 antagonist is an anti-C5 antibody and the frequency of administration of the C5 antagonist to the human having a reduced C5 concentration is no more frequently than once monthly.

68. The method according to aspect 66, wherein the C5 antagonist is an anti-C5 antibody and the frequency of administration of the C5 antagonist to the human having a reduced C5 concentration is no more frequently than once every two months.

69. The method according to any one of aspects 39 to 68, wherein the complement-associated disorder is selected from the group consisting of paroxysmal nocturnal hemoglobinuria (PNH), atypical hemolytic-uremic syndrome (aHUS), shiga toxin *E. coli*-related hemolytic uremic syndrome (STEC-HUS), dense deposit disease (DDD), C3 nephropathy, myasthenia gravis, neuromyelitis optica, cold agglutinin disease (CAD), antineutrophil cytoplasm antibody (ANCA)-associated vasculitis (AAV), asthma, age-related macular degeneration (AMD), transplant rejection, Goodpasture's syndrome, glomerulonephritis, vasculitis, rheumatoid arthritis, dermatitis, systemic lupus erythematosus (SLE), Guillain-Barre syndrome (GBS), dermatomyositis, psoriasis, Graves' disease, Hashimoto's thyroiditis, type I diabetes, pemphigus, autoimmune hemolytic anemia (AIHA), idiopathic thrombocytopenic purpura (ITP), lupus nephritis, ischemia-reperfusion injury, thrombotic thrombocytopenic purpura (TTP), Pauci-immune vasculitis, epidermolysis bullosa, multiple sclerosis, spontaneous fetal loss, recurrent fetal loss, traumatic brain injury, injury resulting from myocardial infarction, cardiopulmonary bypass and hemodialysis, and hemolysis, elevated liver enzymes, and low platelets (HELLP) syndrome.

70. The method according to any one of aspects 39 to 69, wherein the C5 antagonist is depleted at least in part by antigen-mediated clearance.

71. A method for treating a human afflicted with a complement-associated disorder, the method comprising administering to the human a compound that reduces the serum concentration of complement component C5, wherein the human is also to be treated with a C5 antagonist.

72. The method according to aspect 71, wherein the compound inhibits transcription of a human complement component C5 gene.

73. The method according to aspect 71, wherein the compound inhibits translation of an mRNA encoding human complement component C5.

74. The method according to aspect 71, wherein the compound reduces the stability of an mRNA encoding human complement component C5.

75. The method according to aspect 71, wherein the compound is an siRNA specific for mRNA encoding human complement component C5.

76. The method according to aspect 71, wherein the compound is an antisense nucleic acid complementary to a mRNA encoding human complement component C5.

77. The method according to any one of aspects 71 to 76, wherein the C5 antagonist is selected from the group consisting of: MB12/22, MB12/22-RGD, ARC187, ARC1905, SSL7, and OmCI.

78. The method according to any one of aspects 71 to 76, wherein the C5 antagonist is an antibody, or an antigen-binding fragment thereof, that binds to complement component C5 and inhibits the cleavage of C5 into fragments C5a and C5b.

79. The method according to aspect 78, wherein the antibody or antigen-binding fragment thereof is selected from the group consisting of a polyclonal antibody, a recombinant antibody, a diabody, a chimerized or chimeric antibody, a deimmunized antibody, a fully human antibody, a single chain antibody, a domain antibody, an Fv fragment, an Fd fragment, an Fab fragment, an Fab' fragment, and an $F(ab')_2$ fragment.

80. The method according to aspect 78, wherein the antibody is eculizumab.

81. The method according to aspect 78, wherein the antigen-binding fragment is pexelizumab.

82. The method according to any one of aspects 71 to 81, wherein the compound reduces the serum concentration of C5 by at least 10%.

83. The method according to any one of aspects 71 to 81, wherein the compound reduces the serum concentration of C5 by at least 20%.

84. The method according to any one of aspects 71 to 81, wherein the compound reduces the serum concentration of C5 by at least 40%.

85. The method according to any one of aspects 71 to 84, wherein the compound is chronically administered to the human.

86. The method according to aspect 85, wherein the compound is administered to the human at least once weekly for at least three weeks.

87. The method according to aspect 85, wherein the compound is administered to the human at least once weekly for at least six weeks.

88. The method according to aspect 85, wherein the compound is administered to the human at least once weekly for at least six months.

89. The method according to any one of aspects 71 to 88, wherein the compound is subcutaneously administered to the human.

90. The method according to any one of aspects 71 to 89, wherein the C5 antagonist is chronically administered to the human.

91. The method according to any one of aspects 71 to 90, wherein the C5 antagonist and the compound are both chronically administered to the human.

92. The method according to any one of aspects 71 to 91, wherein the C5 antagonist and the compound are administered to the human using different routes of administration.

93. The method according to any one of aspects 71 to 92, wherein the C5 antagonist and the compound are administered to the human under different dosing schedules.

94. The method according to any one of aspects 71 to 93, wherein the serum half-life of the C5 antagonist is increased at least 2-fold as compared to the half-life in the absence of administering the compound.

95. The method according to any one of aspects 71 to 93, wherein the serum half-life of the C5 antagonist is increased at least 5-fold as compared to the half-life in the absence of administering the compound.

96. The method according to any one of aspects 71 to 93, wherein the serum half-life of the C5 antagonist is increased at least 10-fold as compared to the half-life in the absence of administering the compound.

97. The method according to any one of aspects 71 to 93, wherein the therapeutically effective amount of the C5 antagonist administered to the human having a reduced C5 concentration is less than the therapeutically effective amount of the C5 antagonist required without the reduction in C5 concentration.

98. The method according to any one of aspects 71 to 93, wherein the frequency of administration of the C5 antagonist to the human having a reduced C5 concentration is less often than the frequency of administration of the C5 antagonist to the human required without the reduction in C5 concentration.

99. The method according to aspect 98, wherein the C5 antagonist is an anti-C5 antibody and the frequency of administration of the C5 antagonist to the human having a reduced C5 concentration is no more frequently than once monthly.

100. The method according to aspect 98, wherein the C5 antagonist is an anti-C5 antibody and the frequency of administration of the C5 antagonist to the human having a reduced C5 concentration is no more frequently than once every two months.

101. The method according to any one of aspects 71 to 100, wherein the complement-associated disorder is selected from the group consisting of paroxysmal nocturnal hemoglobinuria (PNH), atypical hemolytic-uremic syndrome (aHUS), shiga toxin *E. coli*-related hemolytic uremic syndrome (STEC-HUS), dense deposit disease (DDD), C3 nephropathy, myasthenia gravis, neuromyelitis optica, cold agglutinin disease (CAD), antineutrophil cytoplasm antibody (ANCA)-associated vasculitis (AAV), asthma, age-related macular degeneration (AMD), transplant rejection, Goodpasture's syndrome, glomerulonephritis, vasculitis, rheumatoid arthritis, dermatitis, systemic lupus erythematosus (SLE), Guillain-Barre syndrome (GBS), dermatomyositis, psoriasis, Graves' disease, Hashimoto's thyroiditis, type I diabetes, pemphigus, autoimmune hemolytic anemia (AIHA), idiopathic thrombocytopenic purpura (ITP), lupus nephritis, ischemia-reperfusion injury, thrombotic thrombocytopenic purpura (TTP), Pauci-immune vasculitis, epidermolysis bullosa, multiple sclerosis, spontaneous fetal loss, recurrent fetal loss, traumatic brain injury, injury resulting from myocardial infarction, cardiopulmonary bypass and hemodialysis, and hemolysis, elevated liver enzymes, and low platelets (HELLP) syndrome.

102. The method according to any one of aspects 71 to 101, wherein the C5 antagonist is depleted at least in part by antigen-mediated clearance.

## Claims

1. An antibody, or an antigen-binding fragment thereof, that binds to human complement C5 for use in a method of treating paroxysmal nocturnal hemoglobinuria (PNH) or atypical haemolytic-uremic syndrome (aHUS) in a human, the method comprising:

   (i) administering to the human an siRNA specific for mRNA encoding human complement component C5 to thereby reduce the C5 concentration in the serum of the human, followed by;
   (ii) administering to the human the C5 antibody or fragment thereof.

2. An siRNA specific for mRNA encoding human complement component C5 for use in a method of treating paroxysmal nocturnal hemoglobinuria (PNH) or atypical haemolytic-uremic syndrome (aHUS) in a human, the method comprising:

   (i) administering to the human the siRNA to thereby reduce the C5 concentration in the serum of the human, followed by;
   (ii) administering to the human the C5 antibody or fragment thereof.

3. The antibody for use according to claim 1 or the siRNA for use according to claim 2, wherein the C5 antibody, or an antigen-binding fragment thereof, binds to complement components C5 and inhibits the cleavage of C5 into fragments C5a and C5b.

4. The antibody for use according to claim 1 or 3 or the siRNA for use according to claim 2 or 3, wherein the antibody is eculizumab.

5. The antibody for use according to claim 1 or 3 or the siRNA for use according to claim 2 or 3, wherein the antigen-binding fragment is pexelizumab.

6. The antibody for use according to any one of claims 1 and 3 to 5 or the siRNA for use according to any one of claims 2 to 5, wherein the siRNA reduces the serum concentration of C5 by at least 10%, at least 20% or at least 40%.

7. The antibody for use according to any one of claims 1 and 3 to 6 or the siRNA for use according to any one of claims 2 to 6, wherein the siRNA and/or the antibody is chronically administered to the human.

8. The antibody or siRNA for use according to claim 7, wherein the siRNA is administered to the human at least once weekly for at least three weeks, for at least six weeks or for at least six months.

9. The antibody for use according to any one of claims 1 and 3 to 8 or the siRNA according to any one of claims 2 to 8, wherein the antibody and the siRNA are administered to the human under different dosing schedules.

**10.** The antibody for use according to any one of claims 1 and 3 to 9 or the siRNA for use according to any one of claims 2 to 9, wherein the serum half-life of the C5 antibody or fragment thereof is increased 2-fold, 5-fold or 10-fold as compared to the half-life in the absence of administering the siRNA.

**11.** The antibody for use according to any one of claims 1 and 3 to 10 or the siRNA for use according to any one of claims 2 to 10, wherein the therapeutically effective amounts of the C5 antibody or fragment thereof administered to the human having a reduced C5 concentration is less than the therapeutically effective amount of the C5 antibody or fragment thereof required without the reduction in C5 concentration.

**12.** The antibody for use according to any one of claims 1 and 3 to 11 or the siRNA for use according to any one of claims 2 to 11, wherein the frequency of administration of the C5 antibody or fragment thereof to the human having a reduced C5 concentration is less often than the frequency of administered of the C5 antibody or fragment thereof to the human required without the reduction in C5 concentration.

**13.** The antibody for use according to any one of claims 1 and 3 to 12 or the siRNA for use according to any one of claims 2 to 12 wherein the C5 antibody or fragment thereof is depleted at least in part by antigen-mediated clearance.

**14.** An antibody, or an antigen-binding fragment thereof, that binds to human complement C5, for use in a method of treating paroxysmal nocturnal hemoglobinuria (PNH) or atypical haemolytic-uremic syndrome (aHUS) in a human, wherein the human has a reduced serum concentration of C5, relative to the normal serum concentration of C5, as the result of the prior administration to the human of an siRNA specific for mRNA encoding human complement component C5 that reduces the serum concentration of C5.

**Fig. 1.**

SEQ ID NO:1 (human C5 cDNA sequence; NCBI Accession No. M57729).

```
   1 ctacctccaa ccatgggcct tttgggaata ctttgttttt taatcttcct ggggaaaacc
  61 tggggacagg agcaaacata tgtcatttca gcaccaaaaa tattccgtgt tggagcatct
 121 gaaaatattg tgattcaagt ttatggatac actgaagcat ttgatgcaac aatctctatt
 181 aaaagttatc ctgataaaaa atttagttac tcctcaggcc atgttcattt atcctcagag
 241 aataaattcc aaaactctgc aatcttaaca atacaaccaa aacaattgcc tggaggacaa
 301 aacccagttt cttatgtgta tttggaagtt gtatcaaagc atttttcaaa atcaaaaaga
 361 atgccaataa cctatgacaa tggatttctc ttcattcata cagacaaacc tgtttatact
 421 ccagaccagt cagtaaaagt tagagtttat tcgttgaatg acgacttgaa gccagccaaa
 481 agagaaactg tcttaacctt catagatcct gaaggatcag aagttgacat ggtagaagaa
 541 attgatcata ttggaattat ctcttttcct gacttcaaga ttccgtctaa tcctagatat
 601 ggtatgtgga cgatcaaggc taaatataaa gaggactttt caacaactgg aaccgcatat
 661 tttgaagtta aagaatatgt cttgccacat tttctgtct caatcgagcc agaatataat
 721 ttcattggtt acaagaactt taagaatttt gaaattacta taaaagcaag atatttttat
 781 aataaagtag tcactgaggc tgacgtttat atcacatttg gaataagaga agacttaaaa
 841 gatgatcaaa aagaaatgat gcaaacagca atgcaaaaca caatgttgat aaatggaatt
 901 gctcaagtca catttgattc tgaaacagca gtcaaagaac tgtcatacta cagtttagaa
 961 gatttaaaca acaagtacct ttatattgct gtaacagtca tagagtctac aggtggattt
1021 tctgaagagg cagaaatacc tggcatcaaa tatgtcctct ctccctacaa actgaatttg
1081 gttgctactc ctcttttcct gaagcctggg attccatatc ccatcaaggt gcaggttaaa
1141 gattcgcttg accagttggt aggaggagtc ccagtaatac tgaatgcaca aacaattgat
1201 gtaaaccaag agacatctga cttggatcca agcaaaagtg taacacgtgt tgatgatgga
1261 gtagcttcct ttgtgcttaa tctcccatct ggagtgacgg tgctggagtt taatgtcaaa
1321 actgatgctc cagatcttcc agaagaaaat caggccaggg aaggttaccg agcaatagca
1381 tactcatctc tcagccaaag ttacctttat attgattgga ctgataacca taaggctttg
1441 ctagtgggag aacatctgaa tattattgtt acccccaaaa gcccatatat tgacaaaata
1501 actcactata attacttgat tttatccaag ggcaaaatta tccattttgg cacgagggag
1561 aaattttcag atgcatctta tcaaagtata aacattccag taacacagaa catggttcct
1621 tcatcccgac ttctggtcta ttatatcgtc acaggagaac agacagcaga attagtgtct
1681 gattcagtct ggttaaatat tgaagaaaaa tgtggcaacc agctccaggt tcatctgtct
1741 cctgatgcag atgcatattc tccaggccaa actgtgtctc ttaatatggc aactggaatg
1801 gattcctggg tggcattagc agcagtggac agtgctgtgt atggagtcca aagaggagcc
1861 aaaaagccct tggaaagagt atttcaattc ttagagaaga gtgatctggg ctgtggggca
1921 ggtggtggcc tcaacaatgc caatgtgttc cacctagctg gacttacctt cctcactaat
1981 gcaaatgcag atgactccca agaaaatgat gaaccttgta aagaaattct caggccaaga
2041 agaacgctgc aaaagaagat agaagaaata gctgctaaat ataaacattc agtagtgaag
2101 aaatgttgtt acgatggagc ctgcgttaat aatgatgaaa cctgtgagca gcgagctgca
2161 cggattagtt tagggccaag atgcatcaaa gctttcactg aatgttgtgt cgtcgcaagc
2221 cagctccgtg ctaatatctc tcataaagac atgcaattgg gaaggctaca catgaagacc
2281 ctgttaccag taagcaagcc agaaattcgg agttattttc cagaaagctg gttgtgggaa
2341 gttcatcttg ttcccagaag aaaacagttg cagtttgccc tacctgattc tctaaccacc
2401 tgggaaattc aaggcattgg catttcaaac actggtatat gtgttgctga tactgtcaag
2461 gcaaaggtgt tcaaagatgt cttcctggaa atgaatatac catattctgt tgtacgagga
2521 gaacagatcc aattgaaagg aactgtttac aactatagga cttctgggat gcagttctgt
2581 gttaaaatgt ctgctgtgga gggaatctgc acttcggaaa gcccagtcat tgatcatcag
2641 ggcacaaagt cctccaaatg tgtgcgccag aaagtagagg gctcctccag tcacttggtg
2701 acattcactg tgcttcctct ggaaattggc cttcacaaca tcaatttttc actggagact
2761 tggtttggaa aagaaatctt agtaaaaaca ttacgagtgg tgccagaagg tgtcaaaagg
2821 gaaagctatt ctggtgttac tttggatcct aggggtattt atggtaccat tagcagacga
2881 aaggagttcc catacaggat acccttagat ttggtcccca aaacagaaat caaaaggatt
```

**Fig. 1. (continued)**

```
2941 ttgagtgtaa aaggactgct tgtaggtgag atcttgtctg cagttctaag tcaggaaggc
3001 atcaatatcc taacccacct ccccaaaggg agtgcagagg cggagctgat gagcgttgtc
3061 ccagtattct atgtttttca ctacctggaa acaggaaatc attggaacat ttttcattct
3121 gacccattaa ttgaaaagca gaaactgaag aaaaaattaa aagaagggat gttgagcatt
3181 atgtcctaca gaaatgctga ctactcttac agtgtgtgga agggtggaag tgctagcact
3241 tggttaacag cttttgcttt aagagtactt ggacaagtaa ataaatacgt agagcagaac
3301 caaaattcaa tttgtaattc tttattgtgg ctagttgaga attatcaatt agataatgga
3361 tctttcaagg aaaattcaca gtatcaacca ataaaattac agggtacctt gcctgttgaa
3421 gcccgagaga acagcttata tcttacagcc tttactgtga ttggaattag aaaggctttc
3481 gatatatgcc ccctggtgaa aatcgacaca gctctaatta aagctgacaa ctttctgctt
3541 gaaaatacac tgccagccca gagcaccttt acattggcca tttctgcgta tgctctttcc
3601 ctgggagata aaactcaccc acagtttcgt tcaattgttt cagctttgaa gagagaagct
3661 ttggttaaag gtaatccacc catttatcgt ttttggaaag acaatcttca gcataaagac
3721 agctctgtac ctaacactgg tacggcacgt atggtagaaa caactgccta tgctttactc
3781 accagtctga acttgaaaga tataaattat gttaacccag tcatcaaatg gctatcagaa
3841 gagcagaggt atggaggtgg ctttattca acccaggaca ccatcaatgc cattgagggc
3901 ctgacggaat attcactcct ggttaaacaa ctccgcttga gtatggacat cgatgtttct
3961 tacaagcata aaggtgcctt acataattat aaaatgacag acaagaattt ccttgggagg
4021 ccagtagagg tgcttctcaa tgatgacctc attgtcagta caggatttgg cagtggcttg
4081 gctacagtac atgtaacaac tgtagttcac aaaaccagta cctctgagga agtttgcagc
4141 ttttatttga aaatcgatac tcaggatatt gaagcatccc actacagagg ctacggaaac
4201 tctgattaca aacgcatagt agcatgtgcc agctacaagc ccagcaggga agaatcatca
4261 tctggatcct ctcatgcggt gatggacatc tccttgccta ctggaatcag tgcaaatgaa
4321 gaagacttaa aagcccttgt ggaaggggtg gatcaactat tcactgatta ccaaatcaaa
4381 gatggacatg ttattctgca actgaattcg attccctcca gtgatttcct ttgtgtacga
4441 ttccggatat ttgaactctt tgaagttggg tttctcagtc ctgccacttt cacagtttac
4501 gaataccaca gaccagataa acagtgtacc atgttttata gcacttccaa tatcaaaatt
4561 cagaaagtct gtgaaggagc cgcgtgcaag tgtgtagaag ctgattgtgg gcaaatgcag
4621 gaagaattgg atctgacaat ctctgcagag acaagaaaac aaacagcatg taaaccagag
4681 attgcatatg cttataaagt tagcatcaca tccatcactg tagaaaatgt tttgtcaag
4741 tacaaggcaa cccttctgga tatctacaaa actggggaag ctgttgctga gaaagactct
4801 gagattacct tcattaaaaa ggtaacctgt actaacgctg agctggtaaa aggaagacag
4861 tacttaatta tgggtaaaga agccctccag ataaaataca atttcagttt caggtacatc
4921 taccctttag attccttgac ctggattgaa tactggccta gagacacaac atgttcatcg
4981 tgtcaagcat ttttagctaa tttagatgaa tttgccgaag atatcttttt aaatggatgc
5041 taaaattcct gaagttcagc tgcatacagt ttgcacttat ggactcctgt tgttgaagtt
5101 cgtttttttg ttttcttctt tttttaaaca ttcatagctg gtcttatttg taaagctcac
5161 tttacttaga attagtggca cttgctttta ttagagaatg atttcaaatg ctgtaacttt
5221 ctgaaataac atggccttgg agggcatgaa gacagatact cctccaaggt tattggacac
5281 cggaaacaat aaattggaac acctcctcaa acctaccact caggaatgtt tgctggggcc
5341 gaaagaacag tccattgaaa gggagtatta caaaaacatg gcctttgctt gaaagaaaat
5401 accaaggaac aggaaactga tcattaaagc ctgagtttgc tttc
```

**Fig. 2.**

SEQ ID NO:2 (human pro-C5 amino acid sequence; NCBI Accession No. NP_001726).

```
 -18 mgllgilcfl iflgktwgqe qtyvisapki frvgaseniv iqvygyteaf datisiksyp
  43 dkkfsyssgh vhlssenkfq nsailtiqpk qlpggqnpvs yvylevvskh fskskrmpit
 103 ydngflfiht dkpvytpdqs vkvrvyslnd dlkpakretv ltfidpegse vdmveeidhi
 163 giisfpdfki psnprygmwt ikakykedfs ttgtayfevk eyvlphfsvs iepeynfigy
 223 knfknfeiti karyfynkvv teadvyitfg iredlkddqk emmqtamqnt mlingiaqvt
 283 fdsetavkel syysledlnn kylyiavtvi estggfseea eipgikyvls pyklnlvatp
 343 lflkpgipyp ikvqvkdsld qlvggvpvtl naqtidvnqe tsdldpsksv trvddgvasf
 403 vlnlpsgvtv lefnvktdap dlpeenqare gyraiayssl sqsylyidwt dnhkallvge
 463 hlniivtpks pyidkithyn ylilskgkii hfgtrekfsd asyqsinipv tqnmvpssrl
 523 lvyyivtgeq taelvsdsvw lnieekcgnq lqvhlspdad ayspgqtvsl nmatgmdswv
 583 alaavdsavy gvqrgakkpl ervfqfleks dlgcgagggl nnanvfhlag ltfltnanad
 643 dsqendepck eilrprrtlq kkieeiaaky khsvvkkccy dgacvnndet ceqraarisl
 703 gprcikafte ccvvasqlra nishkdmqlg rlhmktllpv skpeirsyfp eswlwevhlv
 763 prrkqlqfal pdslttweiq gvgisntgic vadtvkakvf kdvflemnip ysvvrgeqiq
 823 lkgtvynyrt sgmqfcvkms avegictses pvidhqgtks skcvrqkveg ssshlvtftv
 883 lpleiglhni nfsletwfgk eilvktlrvv pegvkresys gvtldprgiy gtisrrkefp
 943 yripldlvpk teikrilsvk gllvgeilsa vlsqeginil thlpkgsaea elmsvvpvfy
1003 vfhyletgnh wnifhsdpli ekqklkkklk egmlsimsyr nadysysvwk ggsastwlta
1063 falrvlgqvn kyveqnqnsi cnsllwlven yqldngsfke nsqyqpiklq gtlpvearen
1123 slyltaftvi girkafdicp lvkidtalik adnfllentl paqstftlai sayalslgdk
1183 thpqfrsivs alkrealvkg nppiyrfwkd nlqhkdssvp ntgtarmvet tayalltsln
1243 lkdinyvnpv ikwlseeqry gggfystqdt inaiegltey sllvkqlrls mdidvsykhk
1303 galhnykmtd knflgrpvev llnddlivst gfgsglatvh vttvvhktst seevcsfylk
1363 idtqdieash yrgygnsdyk rivacasykp sreesssgss havmdislpt gisaneedlk
1423 alvegvdqlf tdyqikdghv ilqlnsipss dflcvrfrif elfevgflsp atftvyeyhr
1483 pdkqctmfys tsnikiqkvc egaackcvea dcgqmqeeld ltisaetrkq tackpeiaya
1543 ykvsitsitv envfvkykat lldiyktgea vaekdseitf ikkvtctnae lvkgrqylim
1603 gkealqikyn fsfryiypld sltwieywpr dttcsscqaf lanldefaed iflngc
```

Europäisches Patentamt
European Patent Office
Office européen des brevets

# EUROPEAN SEARCH REPORT

Application Number
EP 18 21 1716

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/109338 A1 (ALEXION PHARMA INC [US]; MAGRO CYNTHIA [US]) 9 September 2011 (2011-09-09) * Whole doc., in particular p.50, 57; p.23, 43, 47, 48 * ----- | 1-14 | INV. A61K39/395 A61K48/00 C07K16/18 |
| A | ROTHER R P ET AL: "Discovery and development of the complement inhibitor eculizumab for the treatment of paroxysmal nocturnal hemoglobinuria", NATURE BIOTECHNOLOGY, NATURE PUBLISHING GROUP, NEW YORK, NY, US, vol. 25, no. 11, 12 December 2007 (2007-12-12), pages 1256-1264, XP002553743, ISSN: 1087-0156, DOI: 10.1038/NBT1344 [retrieved on 2007-11-07] * the whole document * ----- | 1-14 | |
| A | WO 2007/103549 A2 (ARCHEMIX CORP [US]; EPSTEIN DAVID [US]; KURZ JEFF C [US]) 13 September 2007 (2007-09-13) * the whole document * ----- | 1-14 | TECHNICAL FIELDS SEARCHED (IPC) A61K C07K |
| A | CHENG LING-LI ET AL: "[Effect of C5-siRNA silencing receptor C5 on myocardial ischemia injury in rats]", JOURNAL OF SOUTHERN MEDICAL UNIVERSITY - NAN FANG YI KE DA XUEBAO, NANFANG-YIKE-DAXUE <GUANGZHOU>, CN, vol. 30, no. 6, 1 June 2010 (2010-06-01), pages 1486-1488, XP008170341, ISSN: 1673-4254 * the whole document * ----- -/-- | 1-14 | |

1

The present search report has been drawn up for all claims

| Place of search | Date of completion of the search | Examiner |
|---|---|---|
| Munich | 9 January 2019 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 21 1716

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LACHMANN P J ET AL: "Taking complement to the clinic--has the time finally come?", SCANDINAVIAN JOURNAL OF IMMUNOLOGY JUN 2009, vol. 69, no. 6, June 2009 (2009-06), pages 471-478, XP002728313, ISSN: 1365-3083 * the whole document * ----- | 1-14 | |
| A | MOLLNES T E ET AL: "Strategies of therapeutic complement inhibition", MOLECULAR IMMUNOLOGY, PERGAMON, GB, vol. 43, no. 1-2, 1 January 2006 (2006-01-01), pages 107-121, XP027899184, ISSN: 0161-5890 [retrieved on 2006-01-01] * the whole document * ----- | 1-14 | |
| | | | TECHNICAL FIELDS SEARCHED (IPC) |

The present search report has been drawn up for all claims

1

| Place of search | Date of completion of the search | Examiner |
|---|---|---|
| Munich | 9 January 2019 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 21 1716

This annex lists the patent family members relating to the patent documents cited in the above-mentioned European search report. The members are as contained in the European Patent Office EDP file on
The European Patent Office is in no way liable for these particulars which are merely given for the purpose of information.

09-01-2019

| Patent document cited in search report | Publication date | Patent family member(s) | Publication date |
|---|---|---|---|
| WO 2011109338 A1 | 09-09-2011 | AU 2011223866 A1 | 27-09-2012 |
| | | BR 112012022214 A2 | 04-07-2017 |
| | | CA 2791962 A1 | 09-09-2011 |
| | | CN 102985106 A | 20-03-2013 |
| | | EP 2542255 A1 | 09-01-2013 |
| | | ES 2615732 T3 | 08-06-2017 |
| | | JP 5852021 B2 | 03-02-2016 |
| | | JP 2013521294 A | 10-06-2013 |
| | | JP 2016026216 A | 12-02-2016 |
| | | JP 2016026217 A | 12-02-2016 |
| | | KR 20130009784 A | 23-01-2013 |
| | | NZ 602219 A | 31-10-2014 |
| | | RU 2012141556 A | 10-04-2014 |
| | | SG 183541 A1 | 30-10-2012 |
| | | US 2013064820 A1 | 14-03-2013 |
| | | US 2015174243 A1 | 25-06-2015 |
| | | WO 2011109338 A1 | 09-09-2011 |
| WO 2007103549 A2 | 13-09-2007 | AU 2007223796 A1 | 13-09-2007 |
| | | BR PI0708588 A2 | 07-06-2011 |
| | | CA 2643951 A1 | 13-09-2007 |
| | | CA 3009846 A1 | 13-09-2007 |
| | | CA 3009854 A1 | 13-09-2007 |
| | | CN 104623692 A | 20-05-2015 |
| | | DK 1991275 T3 | 08-12-2014 |
| | | DK 2596807 T3 | 07-03-2016 |
| | | EP 1991275 A2 | 19-11-2008 |
| | | EP 2596807 A1 | 29-05-2013 |
| | | EP 3034094 A1 | 22-06-2016 |
| | | EP 3360582 A1 | 15-08-2018 |
| | | ES 2527695 T3 | 28-01-2015 |
| | | ES 2562423 T3 | 04-03-2016 |
| | | HK 1127281 A1 | 25-09-2015 |
| | | HK 1181308 A1 | 12-08-2016 |
| | | HU E026496 T2 | 28-06-2016 |
| | | IL 193828 A | 31-03-2015 |
| | | JP 5406534 B2 | 05-02-2014 |
| | | JP 5746232 B2 | 08-07-2015 |
| | | JP 2009529058 A | 13-08-2009 |
| | | JP 2013067661 A | 18-04-2013 |
| | | JP 2014172910 A | 22-09-2014 |
| | | KR 20080100388 A | 17-11-2008 |
| | | KR 20140072219 A | 12-06-2014 |
| | | KR 20150017388 A | 16-02-2015 |
| | | NZ 571791 A | 30-03-2012 |
| | | PT 1991275 E | 03-02-2015 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 18 21 1716

This annex lists the patent family members relating to the patent documents cited in the above-mentioned European search report. The members are as contained in the European Patent Office EDP file on
The European Patent Office is in no way liable for these particulars which are merely given for the purpose of information.

09-01-2019

| Patent document cited in search report | Publication date | Patent family member(s) | Publication date |
|---|---|---|---|
| | | PT 2596807 E | 18-03-2016 |
| | | RU 2008139901 A | 20-04-2010 |
| | | RU 2012153978 A | 20-06-2014 |
| | | SG 172686 A1 | 28-07-2011 |
| | | SG 2014011290 A | 27-06-2014 |
| | | SG 10201706440R A | 28-09-2017 |
| | | SI 1991275 T1 | 31-03-2015 |
| | | SI 2596807 T1 | 31-03-2016 |
| | | US 2009269356 A1 | 29-10-2009 |
| | | US 2017145416 A1 | 25-05-2017 |
| | | WO 2007103549 A2 | 13-09-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61806687 A
- WO 2010151526 A
- WO 2005074607 A
- WO 2010054403 A
- US 20030166282 A
- US 20030143204 A
- US 20040038278 A
- US 20030224432 A
- US 4987071 A
- US 5116742 A
- US 5093246 A
- WO 2010136311 A
- US 6355245 B
- WO 2004007553 A
- WO 2010015608 A
- US 20070048248 A
- WO 9823289 A
- WO 9734631 A
- US 6277375 B
- WO 9315199 A
- WO 9315200 A
- WO 0177137 A
- EP 413622 A
- US 20110111406 A
- US 20080241223 A
- US 5501856 A
- US 4863457 A
- US 3710795 A
- EP 488401 A
- EP 430539 A
- WO 04026380 A
- WO 04024156 A
- WO 0178693 A
- US 20050271660 A
- US 20090110679 A
- US 6302855 B
- US 5308341 A
- US 6192891 B
- US 6277099 B
- US 6200296 B
- US 6146361 A
- US 7556615 B
- US 20070172483 A


### Non-patent literature cited in the description


- The Merck Manual
- **MÜLLER-EBERHARD.** *Ann Rev Biochem,* 1988, vol. 57, 321-347
- **MEDICUS et al.** *J Exp Med,* 1976, vol. 144, 1076-1093
- **FEARON et al.** *J Exp Med,* 1975, vol. 142, 856-863
- **ISENMAN et al.** *J Immunol,* 1980, vol. 124, 326-331
- **SCHREIBER et al.** *Proc Natl Acad Sci USA,* 1978, vol. 75, 3948-3952
- **SISSONS et al.** *Proc Natl Acad Sci USA,* 1980, vol. 77, 559-562
- **COOPER et al.** *J Exp Med,* 1970, vol. 132, 775-793
- **HOLERS et al.** *Immunological Reviews,* 2008, vol. 223, 300-316
- **ROTHER T.** *Nature Biotechnology,* 2007, vol. 25 (11), 1256-1264
- **WANG et al.** *Proc. Natl. Acad. Sci. USA,* 1996, vol. 93, 8563-8568
- **WANG et al.** *Proc. Natl. Acad. Sci. USA,* 1995, vol. 92, 8955-8959
- **RINDER et al.** *J. Clin. Invest.,* 1995, vol. 96, 1564-1572
- **KROSHUS et al.** *Transplantation,* 1995, vol. 60, 1194-1202
- **HOMEISTER et al.** *J. Immunol.,* 1993, vol. 150, 1055-1064
- **WEISMAN et al.** *Science,* 1990, vol. 249, 146-151
- **AMSTERDAM et al.** *Am. J. Physiol.,* 1995, vol. 268, H448-H457
- **RABINOVICI et al.** *J Immunol,* 1992, vol. 149, 1744-1750
- **HILLMEN et al.** *N Engl J Med,* 2004, vol. 350 (6), 552
- **RAWAL ; PANGBURN.** *J Immunol,* 2001, vol. 166 (4), 2635-2642
- **SISSONS et al.** *Clin Invest,* 1977, vol. 59, 704-715
- **HILL et al.** *Blood,* 2005, vol. 106 (7), 2559
- **CLEMENS et al.** *Proc. Natl. Acad. Sci. USA,* 2000, vol. 97, 6499-6503
- **BILLY et al.** *Proc. Natl. Acad. Sci. USA,* 2001, vol. 98, 14428-14433

- **ELBASHIR et al.** *Nature,* 2001, vol. 411, 494-8
- **YANG et al.** *Proc. Natl. Acad. Sci. USA,* 2002, vol. 99, 9942-9947
- Antisense RNA and DNA. Cold Spring Harbor Laboratory, 1988
- **HASELHOFF ; GERLACH.** *Nature,* 1988, vol. 334, 585-59
- **HELENE.** *Anticancer Drug D,* 1991, vol. 6, 569-84
- **HELENE.** *Ann. NY. Acad. Sci.,* 1992, vol. 660, 27-36
- **MAHER.** *Bioassays,* 1992, vol. 14, 807-15
- *JAm Chem Soc,* 1998, vol. 120, 8565-8566
- **HAGEMANN et al.** *J Biol Chem,* 2008, vol. 283 (12), 7763-75
- **ZUIDERWEG et al.** *Biochemistry,* 1989, vol. 28 (1), 172-85
- **FREDSLUND et al.** *Nat. Immunol.,* 2008, vol. 9, 753-760
- **LAURSEN et al.** *EMBO J.,* 2011, vol. 30, 606-616
- **HAVILAND et al.** *J. Immunol.,* 1991, vol. 146, 362-368
- **ULRICH.** *Handb Exp Pharmacol.,* 2006, vol. 173, 305-326
- **KAPLAN.** *Curr Opin Investig Drugs,* 2002, vol. 3 (7), 1017-23
- **HILL.** *Clin Adv Hematol Oncol,* 2005, vol. 3 (11), 849-50
- **ROTHER et al.** *Nature Biotechnology,* 2007, vol. 25 (11), 1256-1488
- **WHISS.** *Curr Opin Investig Drugs,* 2002, vol. 3 (6), 870-7
- **PATEL et al.** *Drugs Today (Barc),* 2005, vol. 41 (3), 165-70
- **THOMAS et al.** *Mol Immunol.,* 1996, vol. 33 (17-18), 1389-401
- **MOONGKARNDI et al.** *Immunobiol,* 1982, vol. 162, 397
- **MOONGKARNDI et al.** *Immunobiol,* 1983, vol. 165, 323
- **MOLLNES et al.** *Scand J Immunol,* 1988, vol. 28, 307-312
- **ALI et al.** *J Biol Chem,* 1999, vol. 274, 24066-24073
- **HEY et al.** *TRENDS Biotechnol,* 2005, vol. 23 (10), 514-522
- **HEPBURN et al.** *J Biol Chem,* 2007, vol. 282, 8292-8299
- **SOLTYS et al.** *Ann Neurol,* 2009, vol. 65, 67-75
- **LAURSEN et al.** *Proc. Natl. Acad. Sci.,* 2010, vol. 107, 3681-3686
- Experimental Immunochemistry. Springfield, 1961, vol. 135-240, 135-139
- **ISENMAN et al.** *J Immunol.,* 1980, vol. 124 (1), 326-31
- **THOMAS et al.** *Mol. Immunol.,* 1996, vol. 33 (17-18), 1389-401
- **EVANS et al.** *Mol. Immunol.,* 1995, vol. 32 (16), 1183-95
- **WARD ; ZVAIFLER.** *J Clin Invest.,* 1971, vol. 50 (3), 606-16
- **WURZNER et al.** *Complement Inflamm.,* 1991, vol. 8, 328-340
- **HINTON et al.** *J Immunol,* 2006, vol. 176, 346-356
- **VAN GURP et al.** *Am J Transplantation,* 2008, vol. 8 (8), 1711-1718
- **HANOUSKA et al.** *Clin Cancer Res,* 2007, vol. 13 (2), 523-531
- **HETHERINGTON et al.** *Antimicrobial Agents and Chemotherapy,* 2006, vol. 50 (10), 3499-3500
- **APPEL et al.** *J Am Soc Nephrol,* 2005, vol. 16, 1392-1404
- **HOLERS.** *Immunological Reviews,* 2008, vol. 223, 300-316
- **HOLERS ; THURMAN.** *Molecular Immunology,* 2004, vol. 41, 147-152
- **PARK et al.** *Anesth Analg,* 1999, vol. 99 (1), 42-48
- **TOFUKUJI et al.** *J Thorac Cardiovasc Surg,* 1998, vol. 116 (6), 1060-1068
- **SCHMID et al.** *Shock,* 1997, vol. 8 (2), 119-124
- **BLESS et al.** *Am J Physiol,* 1999, vol. 276 (1), L57-L63
- **PETKOVA et al.** *Int Immunology,* 2006, vol. 18 (12), 1759-1769
- **OIAO et al.** *Proc Natl Acad Sci USA,* 2008, vol. 105 (27), 9337-9342
- **ROOPENIAN et al.** *Methods Mol Biol,* 2010, vol. 602, 93-104